# EUROPEAN PATENT SPECIFICATION

(11) **EP 2 166 974 B1**
(45) Date of publication and mention of the grant of the patent: **23.12.2015**
(21) Application number: 08768242.3
(22) Date of filing: 06.06.2008
(51) Int. Cl.: A61B 19/00, A61B 17/34, A61N 1/372, A61N 1/36

(54) **MRI-GUIDED MEDICAL INTERVENTIONAL SYSTEMS**
MRT-GEFÜHRTE MEDIZINISCHE INTERVENTIONSSYSTEME
SYSTÈMES D'INTERVENTION MÉDICALE GUIDÉS PAR IMAGERIE PAR RÉSONANCE MAGNÉTIQUE (IRM)

(30) Priority: 07.06.2007 US 933641 P; 24.09.2007 US 974821 P
(43) Date of publication of application: 31.03.2010
(73) Proprietor: MRI INTERVENTIONS, INC., Irvine, CA 92618 (US)
(72) Inventor: SAYLER, David, John, Portland, Oregon 97231 (US); MAZZEI, Raffaele, Encinitas, California 92024 (US); JENKINS, Kimble, L., Memphis, Tennessee 38111 (US); PIFERI, Peter, Orange, California 92867 (US)
(74) Representative: Uno, Jennifer Elizabeth Hayes
(86) International application number: PCT/US2008/007169
(87) International publication number: WO 2008/153975

(56) References cited:
- WO-A-2004/058086
- WO-A-2006/081409
- US-A1- 2002 052 610

## Description

### FIELD OF THE INVENTION

The present invention relates generally to medical systems and, more particularly, to in vivo medical systems.

### BACKGROUND

Deep Brain Stimulation (DBS) is becoming an acceptable therapeutic modality in neurosurgical treatment of patients suffering from chronic pain, Parkinson's disease or seizure, and other medical conditions. Other electro-stimulation therapies have also been carried out or proposed using internal stimulation of the sympathetic nerve chain and/or spinal cord, etc. One example of a prior art DBS system is the Activa® system from Medtronic, Inc. The Activa® system includes an implantable pulse generator stimulator that is positioned in the chest cavity of the patient and a lead with axially spaced apart electrodes that is implanted with the electrodes disposed in neural tissue. The lead is tunneled subsurface from the brain to the chest cavity connecting the electrodes with the pulse generator. These leads can have multiple exposed electrodes at the distal end that are connected to conductors which run along the length of the lead and connect to the pulse generator placed in the chest cavity.

It is believed that the clinical outcome of certain medical procedures, particularly those using DBS, may depend on the precise location of the electrodes that are in contact with the tissue of interest. For example, to treat Parkinson's tremor, presently the DBS probes are placed in neural tissue with the electrodes transmitting a signal to the thalamus region of the brain. DBS stimulation leads are conventionally implanted during a stereotactic surgery, based on pre-operative MRI and CT images. These procedures can be long in duration and may have reduced efficacy as it has been reported that, in about 30% of the patients implanted with these devices, the clinical efficacy of the device/procedure is less than optimum. Notwithstanding the above, there remains a need for alternative MRI-guided interventional tools for DBS, as well as for other interventional medical procedures.

US 2002/052610 discloses a device and method for targeting and placement of an electrode into the brain or other body organ to treat severe tremor or other neurological disorders.

### SUMMARY

In view of the above, MRI-guided interventional systems and methods are provided. Embodiments of the present invention provide devices and systems for highly localized placement and/or delivery of diagnostic or therapeutic devices or substances.

According to the present invention, there is provided an MRI-guided interventional system as described in claim 1.

According to embodiments of the present invention, an MRI-guided interventional system includes a frame with a cooperating targeting cannula. The frame is configured to be secured to the body of a patient, and is configured to translate and rotate such that the targeting cannula can be positioned to a desired intrabody trajectory. The frame may include one or more MRI-visible fiducial markers that allow frame location/orientation to be determined within an MRI image.

Embodiments of the present invention may be particularly suitable for placing neuro-modulation leads, such as Deep Brain Stimulation ("DBS") leads, implantable parasympathetic or sympathetic nerve chain leads and/or CNS stimulation leads, as well as other devices within the brain.

Embodiments of the present invention may be suitable for a number of interventional procedures in many locations inside the body including, but not limited to, brain, cardiac, spinal, urethral, and the like. Embodiments of the present invention may be suitable for a number of MRI-guided drug delivery procedures, MRI-guided ablation procedures, etc.

A plurality of user-activatable actuators are operably connected to the frame and are configured to translate and rotate the frame relative to the body of a patient so as to position the targeting cannula to a desired intrabody trajectory. In some embodiments, the actuators are dials or thumbscrew-type devices that allow manual manipulation thereof. In other embodiments, the actuators are manipulated remotely using remote controls and cables.

The targeting cannula includes an axially-extending guide bore therethrough that is configured to guide placement of an interventional device in vivo. Various instrumentation and equipment (e.g., stimulation leads, ablation probes or catheters, injection or fluid delivery devices, biopsy needles, extraction tools, etc.) can be inserted through the targeting cannula to execute diagnostic and/or surgical procedures.

According to some embodiments of the present invention, the frame includes a base, a yoke movably mounted to the base and that is rotatable about a roll axis, and a platform movably mounted to the yoke and that is rotatable about a pitch axis. The platform includes an X-Y support table that is configured to move in an X-direction and Y-direction relative to the platform. The base has a patient access aperture formed therein, and is configured to be secured to the body of a patient such that the aperture overlies an opening in the body. A roll actuator is operably connected to the yoke and is configured to rotate the yoke about the roll axis. A pitch actuator is operably connected to the platform and is configured to rotate the platform about the pitch axis. An X-direction actuator is operably connected to the platform and is configured to move the X-Y support table in the X-direction. A Y-direction actuator is operably connected to the platform and is configured to move the X-Y support table in the Y-direction.

The base may include a plurality of locations for attachment to a body of a patient via fasteners. In some embodiments, one or more attachment locations may include multiple adjacent apertures configured to receive a fastener therethrough. For embodiments where the frame is configured to be attached to the skull of a patient, the base can be configured to be secured to the skull of a patient such that the patient access aperture overlies a burr hole formed in the patient skull.

According to some embodiments of the present invention, the yoke includes a pair of spaced apart arcuate arms. The platform engages and moves along the yoke arcuate arms when rotated about the pitch axis. The base includes at least one arcuate arm. The yoke engages and moves along the base arcuate arm when rotated about the roll axis.

According to some embodiments of the present invention, at least one of the yoke arcuate arms includes a thread pattern formed in a surface thereof. The pitch actuator includes a rotatable worm with teeth configured to engage the thread pattern. Rotation of the worm causes the platform to rotate about the pitch axis. Similarly, at least one of the base arcuate arms includes a thread pattern formed in a surface thereof. The roll actuator includes a rotatable worm with teeth configured to engage the thread pattern, and wherein rotation of the worm causes the yoke to rotate about the roll axis.

In some embodiments, the actuators are color-coded such that each different actuator has a respective different color. This allows a user to quickly determine which actuator is the correct one for a particular desired movement of the frame.

According to some embodiments of the present invention, an ergonomic remote control unit is provided that allows a user to remotely translate and rotate the frame such that the targeting cannula can be positioned to a desired intrabody trajectory. The remote control unit includes a plurality of position controls. Each control is operably connected to a respective frame actuator by a respective cable. One or more of the position controls can include both "gross" and "fine" adjustments.

Movement of a position control operates a respective actuator via a respective control cable. For example, the remote control unit includes a roll adjustment control, a pitch adjustment control, an X-direction adjustment control, and a Y-direction adjustment control. A roll control cable is operably connected to the roll adjustment control and to the roll actuator. Movement of the roll adjustment control operates the roll actuator via the roll control cable. A pitch control cable is operably connected to the pitch adjustment control and to the pitch actuator. Movement of the pitch adjustment control operates the pitch actuator via the pitch control cable. An X-direction control cable is operably connected to the X-direction control and to the X-direction actuator. Movement of the X-direction adjustment control operates the X-direction actuator via the X-direction control cable. A Y-direction control cable is operably connected to the Y-direction control and to the Y-direction actuator. Movement of the Y-direction adjustment control operates the Y-direction actuator via the Y-direction control cable.

In some embodiments, the roll adjustment control, pitch adjustment control, X-direction adjustment control, and Y-direction adjustment control are manually-operated thumbwheels, and rotation of each thumbwheel by a user causes corresponding axial rotation of a respective control cable and corresponding axial rotation of a respective actuator. In other embodiments, one or more of the roll adjustment control, pitch adjustment control, X-direction adjustment control, and Y-direction adjustment control are electric motor-assisted, rotatable controls.

In some embodiments, locking mechanisms are associated with the remote unit position controls, and are configured to prevent user operation of the controls when in a locked position.

In some embodiments, each control cable has a geometrically shaped rigid end that is configured to removably engage a free end of a respective actuator. Each control cable rigid end may have a shape that is different from the other control cable rigid ends such that each control cable free end can only removably engage one of the respective actuator free ends. Each control cable includes a flexible elastomeric collar that is configured to surround a respective actuator free end and to maintain engagement of a cable end to a respective actuator free end. Each flexible collar can be rolled or folded back then released to cover and conformably compress against an actuator free end to hold the end of the cable in position; then the collar can be pushed back to easily release the cable from an actuator free end.

According to some embodiments, a safety lanyard may be used to connect the remote control module to a rigid object, such as a patient support frame or head coil (or even the gantry or gantry housing) to prevent over extension of the cables or unwanted adjustments to the trajectory.

According to some embodiments, a drape is provided that is configured to be positioned near the body of a patient within a magnet of an MRI scanner. The drape includes a pocket that is configured to removably receive the remote control unit therein. The drape also includes one or more apertures through which the cables extend from the remote control unit to the frame.

According to some embodiments of the present invention, a camera and/or video imaging device is removably secured to the frame via a bracket. The bracket includes a sleeve that is configured to slidably receive the imaging device therein.

An elongated tubular member extends through the platform and yoke and is secured to the X-Y table of the frame. The targeting cannula is slidably secured within the tubular member and is movable between extended and retracted positions. The targeting cannula is configured to translate in response to translational movement of the X-Y support table and to rotate in response to rotational movement of the yoke and platform to define different axial trajectories extending through the patient access aperture of the base. The tubular member is configured to lock the targeting cannula in an extended position and in a retracted position.

A depth stop is removably secured within a proximal end of the tubular member. The depth stop receives a sheath therein, and is configured to limit the distance that the sheath can extend into the body of a patient. The sheath is configured to receive an elongated interventional device (e.g., imaging probe, stimulation lead, ablation device, injection device, etc.). In some embodiments, the sheath is removable. A locking mechanism is removably secured to the depth stop and is configured to prevent axial movement of an elongated interventional device extending through the sheath.

According to some embodiments of the present invention, an MRI-guided interventional system includes a frame with a cooperating targeting cannula that has a guide bore therethrough that is configured to guide placement of an interventional device in vivo. The frame is configured to rotate such that the targeting cannula can be positioned to a desired intrabody trajectory. The frame includes a base having a patient access aperture formed therein, wherein the base is configured to be secured to the body of a patient; a yoke movably mounted to the base and rotatable about a roll axis; and a platform movably mounted to the yoke and rotatable about a pitch axis. A plurality of user-activatable actuators are operably connected to the frame and are configured to rotate the frame relative to the body of the patient so as to position the targeting cannula to a desired intrabody trajectory. In some embodiments, the actuators are color-coded such that each actuator has a respective different color. In some embodiments, the frame includes a roll actuator operably connected to the yoke and configured to rotate the yoke about the roll axis; and a pitch actuator operably connected to the platform and configured to rotate the platform about the pitch axis.

In some embodiments, the system includes a remote control unit comprising a plurality of elongate control devices. Each control device includes first and second elongate rods axially connected at respective first ends via a first cable. The first rod second end is operably connected to a respective actuator via a second cable. Rotational movement of the second end of the second rod operates the actuator via the second cable. Each second cable may have a geometrically shaped rigid end configured to removably engage a free end of a respective actuator.

MRI-guided interventional methods include affixing a frame with a cooperating targeting cannula to the body of a patient, wherein the frame is configured to translate and rotate such that the targeting cannula can be positioned to a desired intrabody access path trajectory. The targeting cannula includes a guide bore therethrough that is configured to guide placement of an interventional device in vivo. The targeting cannula position is adjusted (e.g., rotated about a roll axis, rotated about a pitch axis, and/or translated in X-Y directions) so that the targeting cannula is aligned with the desired access path trajectory while the patient is positioned within a magnetic field associated with an MRI scanner. Once the targeting cannula is repositioned, an interventional device is inserted through the targeting cannula guide bore and into the body of the patient for therapeutic and/or diagnostic purposes. The targeting cannula is movable between retracted and extended positions, and is moved to the extended position and locked in the extended position prior to the adjusting the access path trajectory thereof.

The necessary rotational and translational adjustments required to reposition the targeting cannula to the desired access path trajectory are displayed to a user via a graphical user interface. Both the actual access path trajectory and desired access path trajectory can be displayed, as well. In addition, the user can view the actual trajectory changing as he/she adjusts the position of the targeting cannula. In some embodiments, an indication of when the actual trajectory is aligned with a desired trajectory can be displayed to the user.

### BRIEF DESCRIPTION OF THE DRAWINGS

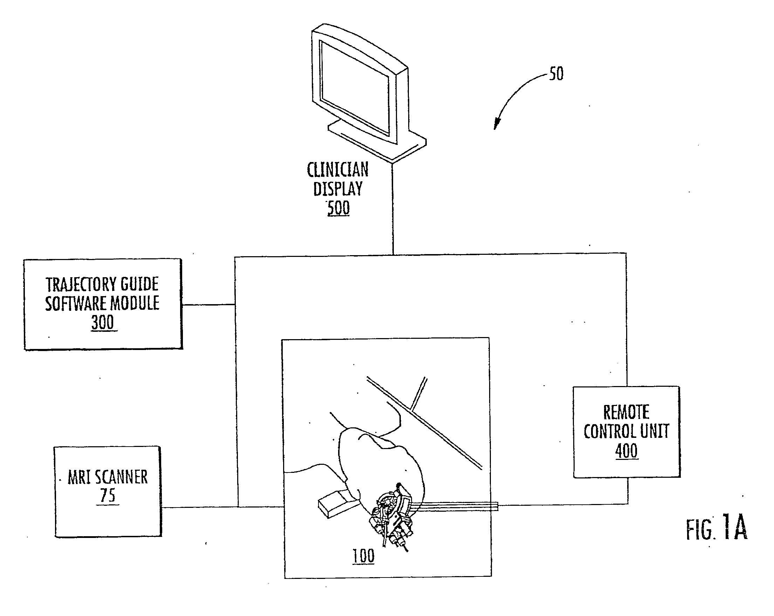

**Fig. 1A** is a block diagram of an MRI-guided interventional system, according to some embodiments of the present invention.
**Fig. 1** **B** illustrates a user interface that displays, and that allows a user to adjust, the trajectory of a targeting cannula, according to some embodiments of the present invention.
**Fig. 2A** is a top perspective view of a burr hole formed in the skull of a patient, and a burr hole ring overlying the burr hole and secured to the skull.
**Fig. 2B** is a top perspective view of a removable centering device positioned on the burr hole ring of **Fig. 1****.**
**Fig. 3A** is a perspective view of a trajectory frame utilized in the MRI-guided interventional system, according to some embodiments of the present invention.
**Figs. 3B-3E** are side view, schematic illustrations of the trajectory frame being secured to the skull of a patient.
**Figs. 4-5** are partial perspective views of the frame of **Fig. 3A** illustrating the base of the frame being positioned on the skull of a patient with the centering device of **Fig. 2B** extending through the patient access aperture.
Fig. **6** illustrates the base secured to the skull of a patient.
**Fig. 7** is an enlarged partial perspective view of the base illustrating an attachment location with a pair of adjacent apertures for receiving fasteners therethrough, according to some embodiments of the present invention.
**Fig. 8A** is a perspective view of the frame of **Fig. 3A** secured to the body (e.g., skull) of a patient, and with the targeting cannula in an extended position.
**Fig. 8B** is a cut-away perspective view of the frame of **Fig. 3A****,** illustrating a targeting cannula according to some embodiments of the present invention.
**Figs. 9** and **10A****-10C** illustrate a remote control unit for remotely controlling the positioning actuators of the frame of **Fig. 3A****,** according to some embodiments of the present invention.
**Fig. 11** is a perspective view of the base of the frame of **Fig. 3A** illustrating fiducial markers associated therewith and illustrating an arcuate arm with a thread pattern formed in a surface thereof that is configured to be engaged by a roll axis actuator, according to some embodiments of the present invention.
**Fig. 12** is a partial perspective view of the frame of **Fig. 3A** illustrating a yoke arcuate arm with a thread pattern formed in a surface thereof that is configured to be engaged by a pitch axis actuator, according to some embodiments of the present invention.
**Figs. 13A-13B** illustrate an optic fiber probe for a video imaging camera mounted to the frame of **Fig. 3A** so as to view a burr hole, according to some embodiments of the present invention.
**Fig. 14** is an enlarged, partial perspective view of the frame of **Fig. 3A** illustrating the targeting cannula locked in an extended position, according to some embodiments of the present invention.
**Fig. 15** is an enlarged, partial perspective view of the frame of **Fig. 3A** illustrating control cables removably engaged with respective actuators, and illustrating flexible elastomeric collars configured to surround respective actuator free ends and to maintain engagement of the cable ends to a respective actuator free end, according to some embodiments of the present invention.
**Fig. 16A** is a partial perspective view of a frame of an MRI-guided interventional system, according to other embodiments of the present invention, and illustrating actuators positioned on a side of the frame and illustrating control cables removably engaged with the respective actuators.
**Fig. 16B** is a partial perspective view of an exemplary prototype actuator illustrating a remote control cable end about to be inserted into a slot in the actuator free end, according to some embodiments of the present invention.
**Fig. 16C** is a partial perspective view of the actuator of **Fig. 16B** with the remote control cable end inserted into the actuator and with an elastomeric collar engaging the free end of the actuator to prevent the cable from being inadvertently removed from the actuator.
**Figs. 16D-16E** are partial perspective views of the actuator of **Fig. 16C** illustrating removal of the elastomeric collar and cable **(****Fig. 16E****)** from the free end of the actuator.
**Fig. 17** illustrates the frame of **Fig. 3A** secured to the skull of a patient and illustrates a desired trajectory for an interventional device, and also illustrates the actual trajectory of the interventional device as oriented by the frame.
**Fig. 18** illustrates the frame of **Fig. 17** after reorientation via manipulation of one or more frame actuators such that the actual trajectory is adjusted to be in alignment with the desired trajectory.
**Fig. 19A** is an enlarged, partial perspective view of the frame of **Fig. 3A** illustrating the X-Y support table, according to some embodiments of the present invention.
**Fig. 19B** schematically illustrates X-Y translation of an X-Y support table and rotational movement of the yoke and platform, according to some embodiments of the present invention.
**Fig. 19C** is partial perspective view of an X-Y support table, according to some embodiments, with elements removed to reveal internal components of an X-direction actuator and Y-direction actuator.
**Fig. 20** illustrates a depth stop with a peel-away sheath inserted therein, according to some embodiments of the present invention.
**Fig. 21** illustrates an imaging probe inserted within the peel-away sheath of **Fig. 20** and with the depth stop advanced to a depth mark on the peel-away sheath, according to some embodiments of the present invention.
**Fig. 22** illustrates the depth stop and probe being inserted into the targeting cannula of the frame of **Fig. 3A****.**
**Fig. 23** illustrates the probe of **Fig. 22** being removed from the peel-away sheath and depth stop.
**Fig. 24** illustrates a lead lock secured to the depth stop of **Fig. 23****.**
**Fig. 25** illustrates a lead being inserted through the lead lock of **Fig. 24** and through the targeting cannula.
**Fig. 26A** is a perspective view of the frame of **Fig. 3A** with the lead of **Fig. 25** inserted into the brain of a patient and with the peel-away sheath being removed.
**Fig. 26B** is an enlarged view of the distal end of the peel-away sheath with the distal end of the lead extending therethrough, prior to removal of the sheath.
**Fig. 27** illustrates a clamp inserted within and attached to the burr hole ring that is configured to prevent the lead from being retracted from the brain as the frame is removed from the skull of the patient.
**Figs. 28A-28G** are side view, schematic illustrations of the trajectory frame illustrating exemplary operation of the device for the insertion of interventional devices within the body of a patient via the targeting cannula.
**Fig. 29** illustrates a drape configured to be positioned adjacent to a patient and that has a pocket configured to removably receive the remote control unit of **Figs. 9** and **10A****-10C.**
**Fig. 30** illustrates a safety lanyard according to some embodiments of the present invention, wherein the safety lanyard is attached to the remote control unit of **Figs. 9** and **10A****-10C** and to a rigid object to prevent inadvertent detachment of the control cables.
**Fig. 31** is a schematic illustration of a patient positioned within an MRI scanner and a user utilizing a remote control apparatus **400** and display monitors to position a targeting cannula.
**Figs. 32A-32C** illustrate a remote control unit for remotely controlling the positioning actuators of the frame of **Fig. 3A****,** according to other embodiments of the present invention.

### DETAILED DESCRIPTION

The present invention now is described more fully hereinafter with reference to the accompanying drawings, in which some embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

Like numbers refer to like elements throughout. In the figures, the thickness of certain lines, layers, components, elements or features may be exaggerated for clarity.
The term "MRI visible" means that a device is visible, directly or indirectly, in an MRI image. The visibility may be indicated by the increased SNR of the MRI signal proximate to the device (the device can act as an MRI receive antenna to collect signal from local tissue) and/or that the device actually generates MRI signal itself, such as via suitable hydro-based coatings and/or fluid (typically aqueous solutions) filled channels or lumens.

The term "MRI compatible" means that a device is safe for use in an MRI environment and/or can operate as intended in an MRI environment, and, as such, if residing within the high-field strength region of the magnetic field, is typically made of a non-ferromagnetic MRI compatible material(s) suitable to reside and/or operate in a high magnetic field environment.

The term "high-magnetic field" refers to field strengths above about 0.5 T, typically above 1.0T, and more typically between about 1.5T and 10T.

The term "targeting cannula" refers to an elongate device, typically having a substantially tubular body that can be oriented to provide positional data relevant to a target treatment site and/or define a desired access path orientation or trajectory. At least portions of a targeting cannula contemplated by embodiments of the invention can be configured to be visible in an MRI image, thereby allowing a clinician to visualize the location and orientation of the targeting cannula in vivo relative to fiducial and/or internal tissue landscape features. Thus, the term "cannula" refers to an elongate device that can be associated with a trajectory frame that attaches to a patient, but does not necessarily enter the body of a patient.

The term "imaging coils" refers to a device that is configured to operate as an MRI receive antenna. The term "coil" with respect to imaging coils is not limited to a coil shape but is used generically to refer to MRI antenna configurations, loopless, looped, etc., as are known to those of skill in the art.
The term "fluid-filled" means that the component includes an amount of the fluid but does not require that the fluid totally, or even substantially, fill the component or a space associated with the component. The fluid may be an aqueous solution, MR contrast agent, or any material that generates MRI signal.

The term "two degrees of freedom" means that the trajectory frame described herein allows for at least translational (swivel or tilt) and rotational movement over a fixed site, which may be referred to as a Remote Center of Motion (RCM).

Embodiments of the present invention can be configured to guide and/or place diagnostic or interventional devices and/or therapies to any desired internal region of the body or object using MRI and/or in an MRI scanner or MRI interventional suite. The object can be any object, and may be particularly suitable for animal and/or human subjects. Some embodiments can be sized and configured to place implantable DBS leads for brain stimulation, typically deep brain stimulation. Some embodiments can be configured to deliver tools or therapies that stimulate a desired region of the sympathetic nerve chain. Other uses inside or outside the brain include stem cell placement, gene therapy or drug delivery for treating physiological conditions. Some embodiments can be used to treat tumors. Some embodiments can be used for RF ablation, laser ablation, cryogenic ablation, etc. In some embodiments the trajectory frame and/or interventional tools can be configured to facilitate high resolution imaging via integral intrabody imaging coils (receive antennas), and/or the interventional tools can be configured to stimulate local tissue, which can facilitate confirmation of proper location by generating a physiologic feedback (observed physical reaction or via fMRI).

Some embodiments can be used to deliver bions, stem cells or other target cells to site-specific regions in the body, such as neurological target and the like. The systems deliver stem cells and/or other cardio-rebuilding cells or products into cardiac tissue, such as a heart wall via a minimally invasive MRI guided procedure, while the heart is beating (i.e., not requiring a non-beating heart with the patient on a heart-lung machine). Examples of known stimulation treatments and/or target body regions are described in U.S. Patent Nos. 6,708,064; 6,438,423; 6,356,786; 6,526,318; 6,405,079; 6,167,311; 6,539,263; 6,609,030 and 6,050,992.

Generally stated, some embodiments of the invention are directed to MRI interventional procedures and provide interventional tools and/or therapies that may be used to locally place interventional tools or therapies in vivo to site-specific regions using an MRI system. The interventional tools can be used to define an MRI-guided trajectory or access path to an in vivo treatment site. Some embodiments of the invention provide interventional tools that can provide positional data regarding location and orientation of a tool in 3-D space with a visual confirmation on an MRI. Embodiments of the invention may provide an integrated system that may allow physicians to place interventional devices/leads and/or therapies accurately and in shorter duration procedures over conventional systems (typically under six hours for DBS implantation procedures, such as between about 1-5 hours).

In some embodiments, MRI can be used to visualize (and/or locate) a therapeutic region of interest inside the brain or other body locations and utilize MRI to visualize (and/or locate) an interventional tool or tools that will be used to deliver therapy and/or to place a chronically implanted device that will deliver therapy. Then, using the three-dimensional data produced by the MRI system regarding the location of the therapeutic region of interest and the location of the interventional tool, the system and/or physician can make positional adjustments to the interventional tool so as to align the trajectory of the interventional tool, so that when inserted into the body, the interventional tool will intersect with the therapeutic region of interest. With the interventional tool now aligned with the therapeutic region of interest, an interventional probe can be advanced, such as through an open lumen inside of the interventional tool, so that the interventional probe follows the trajectory of the interventional tool and proceeds to the therapeutic region of interest. It should be noted that the interventional tool and the interventional probe may be part of the same component or structure. A sheath may optionally form the interventional tool or be used with an interventional probe or tool.

In particular embodiments, using the MRI in combination with local or internal imaging coils and/or MRI contrast material that may be contained at least partially in and/or on the interventional probe or sheath, the location of the interventional probe within the therapeutic region of interest can be visualized on a display or image and allow the physician to either confirm that the probe is properly placed for delivery of the therapy (and/or placement of the implantable device that will deliver the therapy) or determine that the probe is in the incorrect or a non-optimal location. Assuming that the interventional probe is in the proper desired location, the therapy can be delivered and/or the interventional probe can be removed and replaced with a permanently implanted therapeutic device at the same location.

In the event that the physician determines from the MRI image produced by the MRI and the imaging coils, which may optionally be contained in or on the interventional probe, that the interventional probe is not in the proper location, a new therapeutic target region can be determined from the MRI images, and the system can be updated to note the coordinates of the new target region. The interventional probe is typically removed (e.g., from the brain) and the interventional tool can be repositioned so that it is aligned with the new target area. The interventional probe can be reinserted on a trajectory to intersect with the new target region. Although described and illustrated herein with respect to the brain and the insertion of deep brain stimulation leads, it is understood that embodiments of the present invention may be utilized at other portions of the body and for various other types of procedures.

Exemplary embodiments are described below with reference to block diagrams and/or flowchart illustrations of methods, apparatus (systems and/or devices) and/or computer program products. It is understood that a block of the block diagrams and/or flowchart illustrations, and combinations of blocks in the block diagrams and/or flowchart illustrations, can be implemented by computer program instructions. These computer program instructions may be provided to a processor of a general purpose computer, special purpose computer, and/or other programmable data processing apparatus to produce a machine, such that the instructions, which execute via the processor of the computer and/or other programmable data processing apparatus, create means (functionality) and/or structure for implementing the functions/acts specified in the block diagrams and/or flowchart block or blocks.

These computer program instructions may also be stored in a computer-readable memory that can direct a computer or other programmable data processing apparatus to function in a particular manner, such that the instructions stored in the computer-readable memory produce an article of manufacture including instructions which implement the functions/acts specified in the block diagrams and/or flowchart block or blocks.

The computer program instructions may also be loaded onto a computer or other programmable data processing apparatus to cause a series of operational steps to be performed on the computer or other programmable apparatus to produce a computer-implemented process such that the instructions which execute on the computer or other programmable apparatus provide steps for implementing the functions/acts specified in the block diagrams and/or flowchart block or blocks.

Accordingly, exemplary embodiments may be implemented in hardware and/or in software (including firmware, resident software, micro-code, etc.). Furthermore, exemplary embodiments may take the form of a computer program product on a computer-usable or computer-readable storage medium having computer-usable or computer-readable program code embodied in the medium for use by or in connection with an instruction execution system. In the context of this document, a computer-usable or computer-readable medium may be any medium that can contain, store, communicate, propagate, or transport the program for use by or in connection with the instruction execution system, apparatus, or device.

The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM). Note that the computer-usable or computer-readable medium could even be paper or another suitable medium upon which the program is printed, as the program can be electronically captured, via, for instance, optical scanning of the paper or other medium, then compiled, interpreted, or otherwise processed in a suitable manner, if necessary, and then stored in a computer memory.

Computer program code for carrying out operations of data processing systems discussed herein may be written in a high-level programming language, such as Java, AJAX (Asynchronous JavaScript), C, and/or C++, for development convenience. In addition, computer program code for carrying out operations of exemplary embodiments may also be written in other programming languages, such as, but not limited to, interpreted languages. Some modules or routines may be written in assembly language or even micro-code to enhance performance and/or memory usage. However, embodiments are not limited to a particular programming language. It will be further appreciated that the functionality of any or all of the program modules may also be implemented using discrete hardware components, one or more application specific integrated circuits (ASICs), or a programmed digital signal processor or microcontroller.

Embodiments of the present invention will now be described in detail below with reference to the figures. **Fig. 1A** is a block diagram of an MRI-guided interventional system **50,** according to some embodiments of the present invention. The illustrated system **50** includes an MRI scanner **75,** a trajectory frame **100** attached to the body of a patient positioned within a magnetic field of the MRI scanner **75,** a remote control unit **400,** a trajectory guide software module **300,** and a clinician display **500.** The trajectory frame **100** supports a targeting cannula through which various interventional devices may be inserted into the body of a patient. The frame **100** is adjustable such that the targeting cannula is rotatable about a pitch axis, about a roll axis, and such that the targeting cannula can translate in X-Y directions. The frame **100** may be attached to the body of a patient directly or indirectly and may be configured to be attached to various parts of the body.

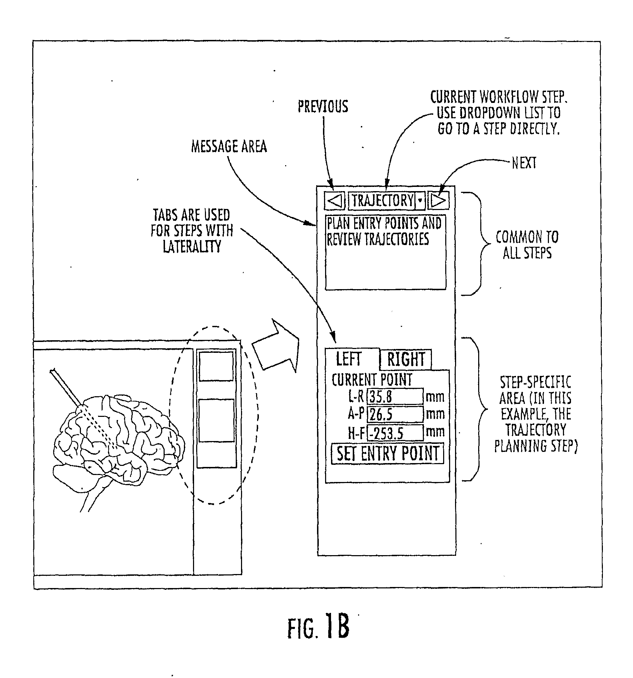

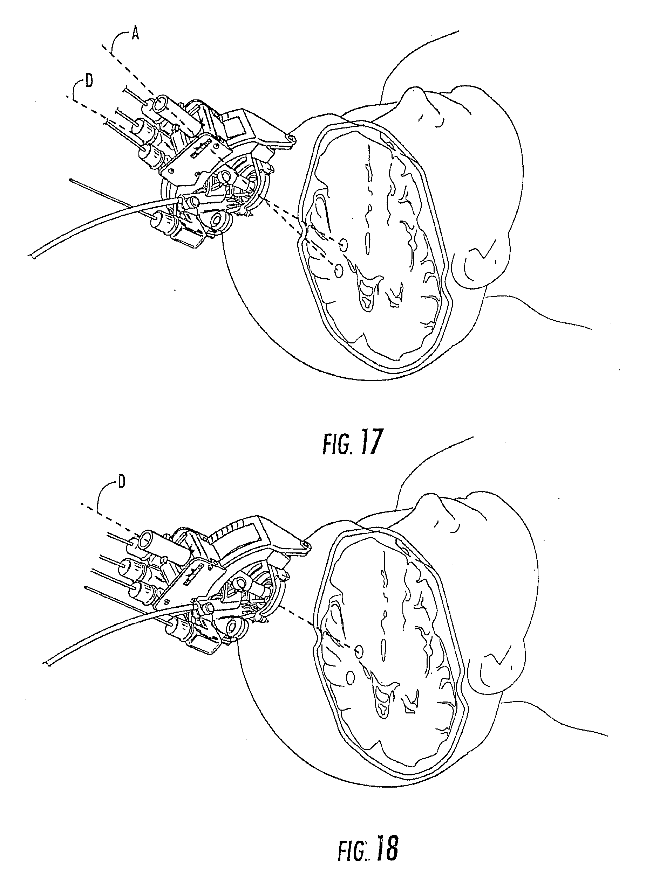

In some embodiments, a remote control unit **400** is provided to allow a user to remotely adjust the position of the targeting cannula. The trajectory guide software module **300** allows a user to define and visualize, via display **500,** a desired trajectory **(D,** **Figs. 17-18****)** into the body of a patient of an interventional device extending through the targeting cannula. The trajectory guide software module **300** also allows the user to visualize and display, via display **500,** an actual trajectory **(A,** **Fig. 17****)** into the body of an interventional device extending through the targeting cannula. The trajectory guide software module **300** displays to the user the necessary positional adjustments (e.g., pitch axis rotation, roll axis rotation, X-Y translation) needed to align the actual trajectory of the targeting cannula with the desired trajectory path **(****Fig. 1 B)****.** In addition, the user can view, via display **500,** the actual trajectory changing as he/she adjusts the position of the targeting cannula. The trajectory guide software module **300** is configured to indicate and display when an actual trajectory is aligned with a desired trajectory.

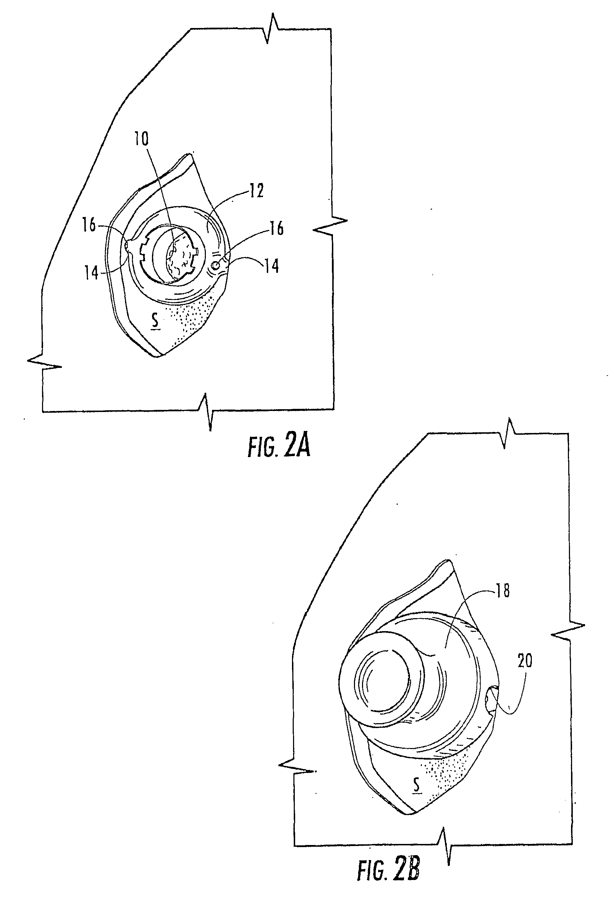

**Fig. 2A** illustrates a burr hole **10** formed in the skull **S** of a patient. A burr hole ring **12** overlies the burr hole **10** and is secured to the skull **S.** The illustrated burr hole ring **12** has a pair of ears **14,** each configured to receive a respective fastener (e.g., screw) therethrough for securing the burr hole ring **12** to the skull. The burr hole ring **12** is secured to the skull **S** via screws **16.** **Fig. 2B** illustrates a removable centering device **18** positioned on the burr hole ring **12.** The centering device **18** includes cut out portions **20** that fit over the ears **14** of the burr hole ring **12.** The function of the centering device **18** is to facilitate centering a trajectory frame **100,** described below, over the burr hole **10.** After the frame **100** is attached to the skull of a patient, the centering device **18** is removed.

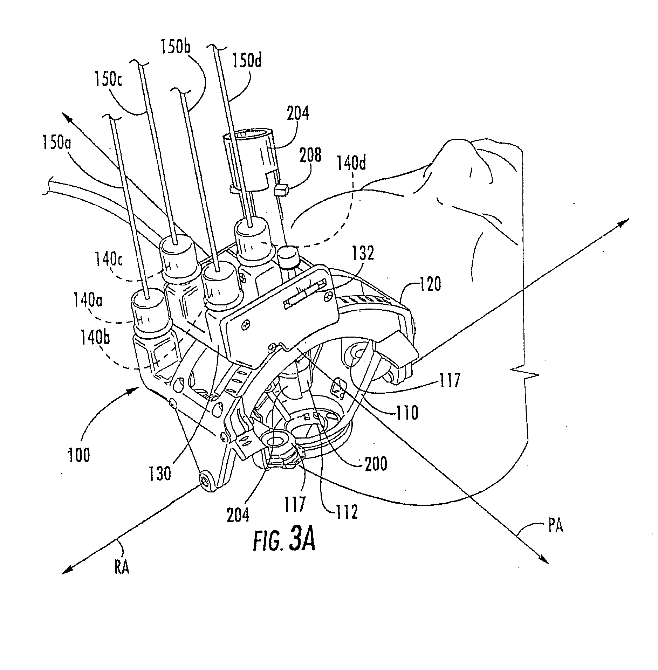

Referring to **Fig. 3A****,** a trajectory frame **100** with a targeting cannula **200** associated therewith is illustrated. The trajectory frame **100** allows for the adjustability (typically at least two degrees of freedom, including rotational and translational) and calibration/fixation of the trajectory of the targeting cannula **200** and/or probe or tool inserted through the targeting cannula **200.** The targeting cannula **200** includes an axially-extending guide bore (not shown) therethrough that is configured to guide the desired therapeutic or diagnostic tool, e.g., intra-brain placement of a stimulation lead (or other type of device) in vivo, as will be described below. Intra-brain placement of devices may include chronically placed devices and acutely placed devices. The trajectory frame **100** may include fiducial markers **117** that can be detected in an MRI to facilitate registration of position in an image.

The illustrated trajectory frame **100** is configured to be mounted to a patient's skull around a burr hole ring **(12,** **Fig. 1****)** and over a burr hole **(10,** **Fig. 1****),** to provide a stable platform for advancing surgical devices, leads, etc. in the brain. The frame **100** includes a base **110,** a yoke, **120,** a platform **130,** and a plurality of actuators **140a-140d.** The base **110** has a patient access aperture **112** formed therein, as illustrated. The base **110** is configured to be secured (directly or indirectly) to the skull of a patient such that the patient access aperture **112** overlies the burr hole **10** in the patient skull. The patient access aperture **112** is centered over the burr hole **10** via the removable centering device **18.** The yoke **120** is movably mounted to the base **110** and is rotatable about a roll axis **RA.** A roll actuator **140a** is operably connected to the yoke **120** and is configured to rotate the yoke **120** about the roll axis **RA,** as will be described in detail below. In some embodiments, the yoke **120** has a range of motion about the roll axis **RA** of about seventy degrees (70°). However, other ranges, greater and lesser than 70°, are possible, e.g., any suitable angle typically between about 10°- 90°, 30°- 90°, etc. The illustrated platform **130** is movably mounted to the yoke **120** and is rotatable about a pitch axis **PA.** In some embodiments, the platform **130** has a range of motion about the pitch axis **PA** of about seventy degrees (70°). However, other ranges, greater and lesser than 70°, are possible, e.g., any suitable angle typically between about 10°- 90°, 30°- 90°, etc.

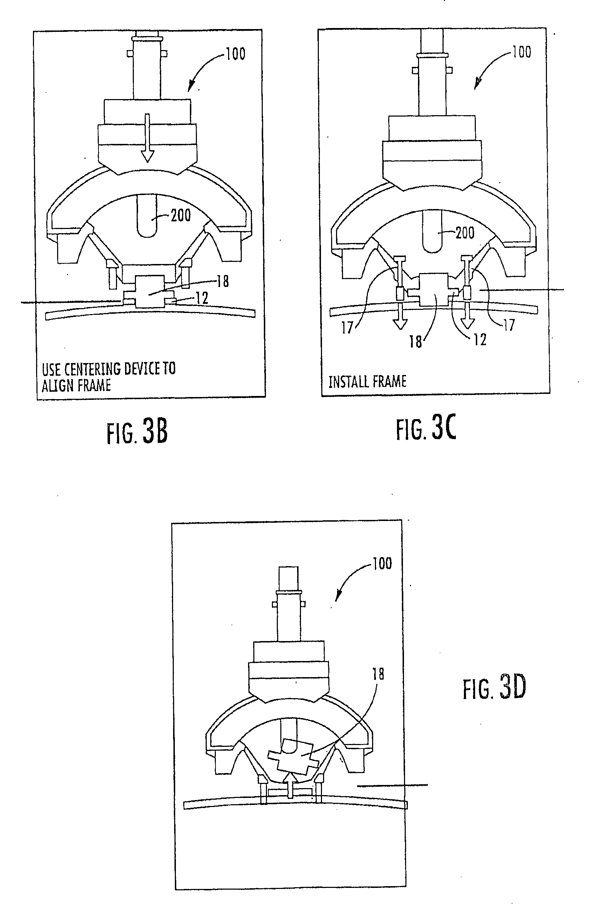

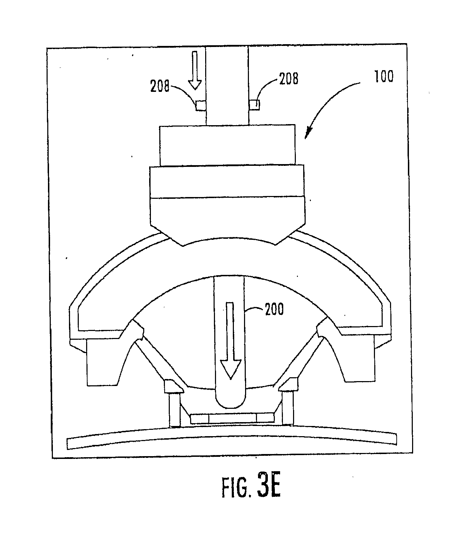

**Figs. 3B-3E** are side view, schematic illustrations of the trajectory frame being secured to the skull of a patient. **Fig. 3B** illustrates use of the centering device **18** to align the frame **100** relative to the burr hole **10.** In **Fig. 3C****,** the frame **100** is secured to the skull with fasteners and such that the patient access aperture **112** in the base **110** is centered around the centering device **18.** In **Fig. 3D****,** the yoke **120** is rotated out of the way such that the centering device **18** can be removed. In **Fig. 3E****,** the targeting cannula **200** is moved to an extended position and locked in the extended position via prongs **208.**

The platform **130** includes an X-Y support table **132** that is movably mounted to the platform **130.** The X-Y support table **132** is configured to move in an X-direction and Y-direction relative to the platform **130.** An X-direction actuator **140c** is operably connected to the platform **130** and is configured to move the X-Y support table **132** in the X-direction. A Y-direction actuator **140d** is operably connected to the platform **130** and is configured to move the X-Y support table **132** in the Y-direction. A pitch actuator **140b** is operably connected to the platform **130** and is configured to rotate the platform **130** about the pitch axis **PA,** as will be described in detail below.

The actuators **140a-140d** are configured to translate and/or rotate the frame. The targeting cannula **200** is configured to translate in response to translational movement of the X-Y support table **132** and to rotate in response to rotational movement of the yoke **120** and platform **130** to define different axial intrabody trajectories extending through the patient access aperture **112** in the frame base **110.**

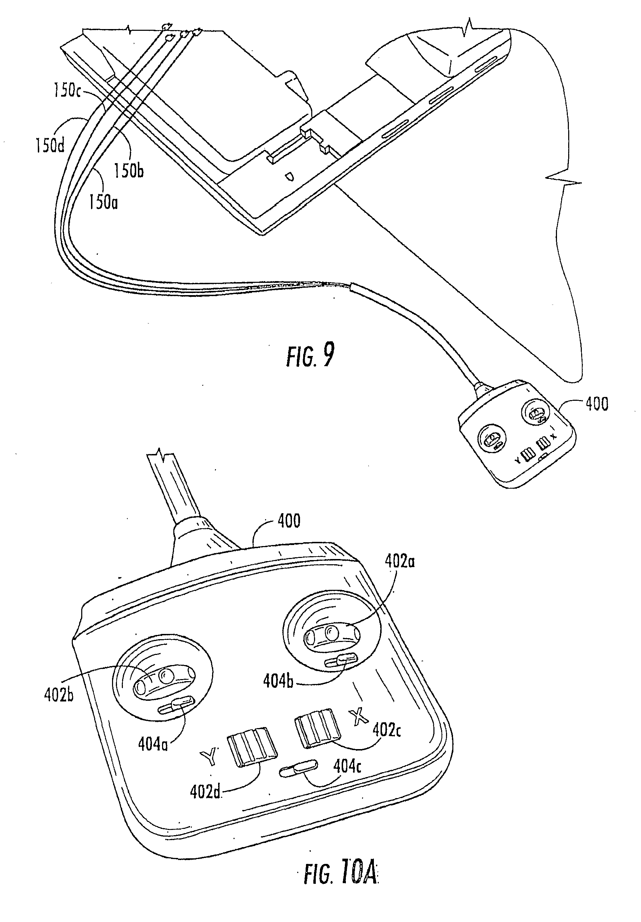

The actuators **140a-140d** may be manually-operated devices, such as thumbscrews, in some embodiments. The thumbscrews can be mounted on the frame **100** or may reside remotely from the frame **100.** A user may turn the actuators **140a-140d** by hand to adjust the position of the frame **100** and, thereby, a trajectory of the targeting cannula **200.** In other embodiments, the actuators **140a-140d** are operably connected to a remote control unit **400 (****Figs. 9-10****)** via a respective plurality of non-ferromagnetic, flexible drive shafts or control cables **150a-150d.** The remote control unit **400** includes a plurality of position controls **402a-402d,** and each cable **150a-150d** is operably connected to a respective position control **402a-402d** and to a respective actuator **140a-140d.** Movement of a position control **402a-402d** operates a respective actuator **140a-140d** via a respective control cable **150a-150d,** as will be described below. The cables **150a-150d** may extend a suitable distance (e.g., between about 1-4 feet, etc.) to allow a clinician to adjust the settings on the trajectory frame **100** without moving a patient and from a position outside the bore of a magnet (where such magnet type is used) associated with an **MRI** scanner.

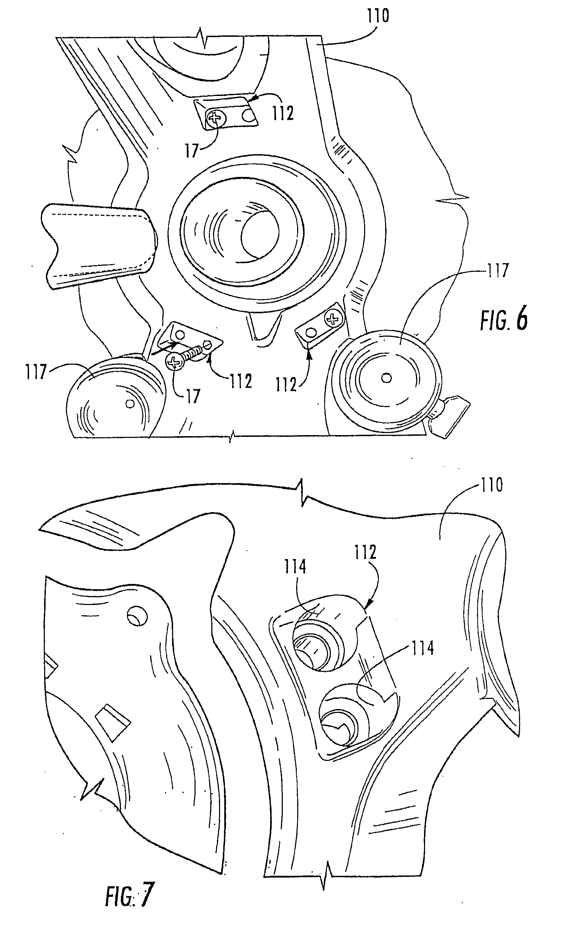

Referring to **Figs. 6-7****,** the base **110** includes a plurality of locations **112** for attaching the base **110** to a skull of a patient via fasteners. Each location may include two or more adjacent apertures **114.** Each aperture **114** is configured to receive a fastener (e.g., a screw, rod, pin, etc.) therethrough that is configured to secure the base **110** to the skull of a patient.

The base **110** also includes MRI-visible fiducial markers **117** that allow the location/orientation of the frame **100** to be determined within an MRI image during an MRI-guided procedure. In the illustrated embodiment, the fiducial markers **117** have a torus or "doughnut" shape and are spaced apart. However, fiducial markers having various shapes and positioned at various locations on the frame **100** may be utilized.

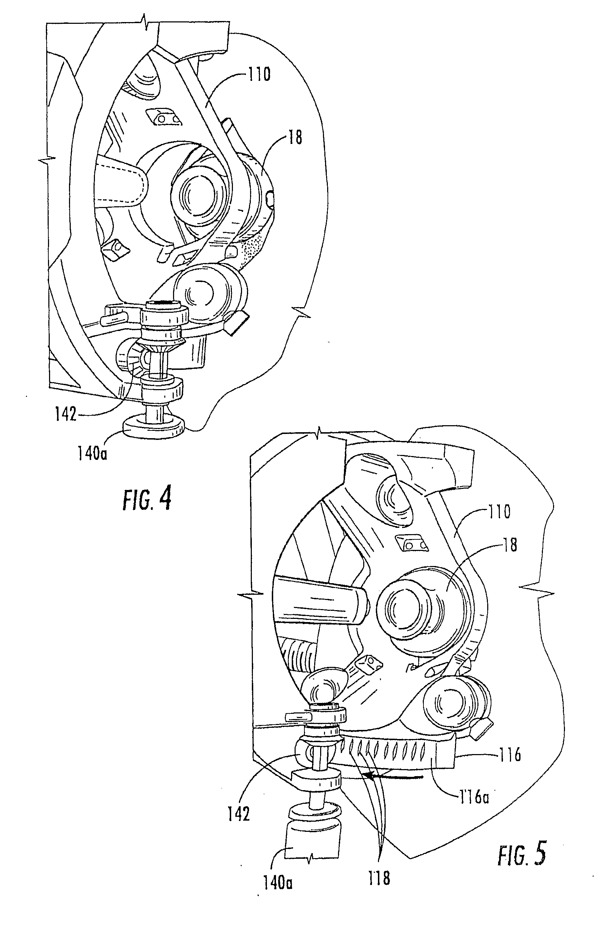

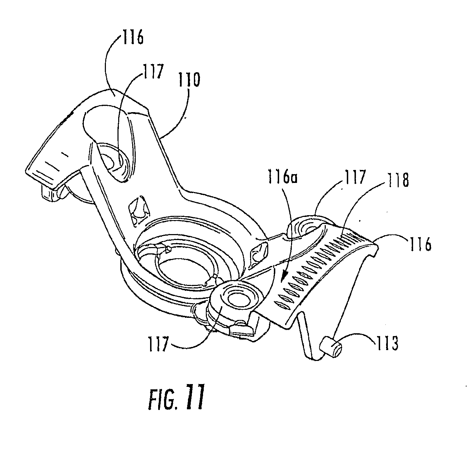

The base **110** also includes a pair of spaced apart arcuate arms **116,** as illustrated in **Fig. 11****.** The yoke **120** is pivotally attached to pivot points **113** for rotation about the roll axis **RA.** The yoke **120** engages and moves along the base arcuate arms **116** when rotated about the roll axis **RA.** In the illustrated embodiment, one of the base arcuate arms **116** includes a thread pattern **118** formed in (e.g., embossed within, machined within, etc.) a surface **116a** thereof. However, in other embodiments, both arms **116** may include respective thread patterns. The roll actuator **140a** includes a rotatable worm **142** with teeth that are configured to engage the thread pattern **118,** as illustrated in **Fig. 5****.** As the worm **142** is rotated, the teeth travel along the thread pattern **118** in the arcuate arm surface **116a.** Because the base **110** is fixed to a patient's skull, rotation of the roll actuator worm **142** causes the yoke **120** to rotate about the roll axis **RA** relative to the fixed base **110.** Rotation about roll axis **RA** is illustrated in **Figs. 4-****5.** For example, in **Fig. 5****,** the yoke **120** is rotated about the roll axis **RA** sufficiently to allow removal of the centering device **18.**

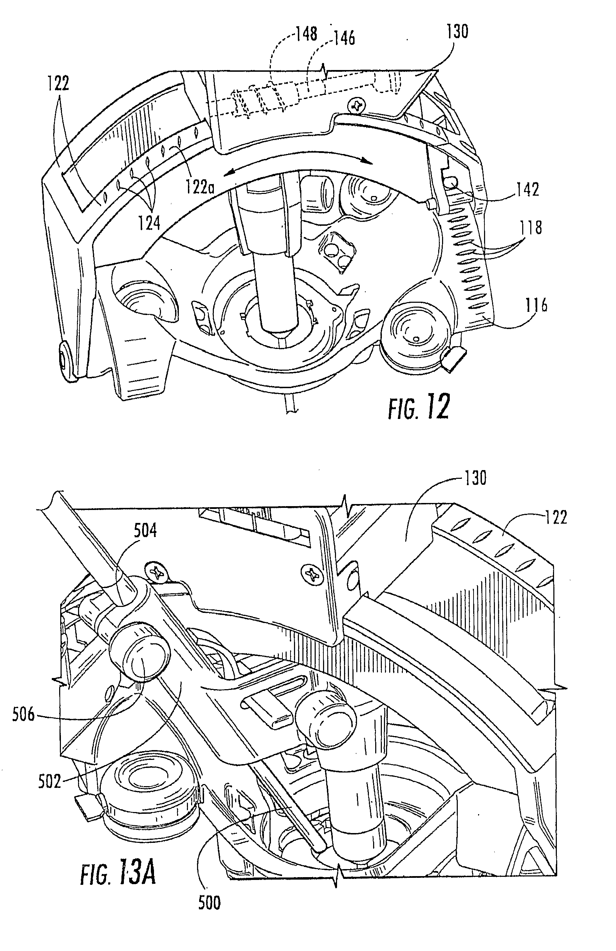

Referring to **Fig. 12****,** the yoke **120** includes a pair of spaced apart upwardly extending, arcuate arms **122.** The platform **130** engages and moves along the yoke arcuate arms **122** when rotated about the pitch axis **PA.** In the illustrated embodiment, one of the yoke arcuate arms **122** includes a thread pattern **124** formed in (e.g., embossed within, machined within, etc.) a surface **122a** thereof. However, in other embodiments, both arms **122** may include respective thread patterns. The pitch actuator **140b** includes a rotatable worm **146** with teeth **148** that are configured to engage the thread pattern **124.** As the worm **146** is rotated, the teeth **148** travel along the thread pattern **124** in the arcuate arm surface **122a.** Because the base **110** is fixed to a patient's skull, rotation of the pitch actuator worm **146** causes the platform **130** to rotate about the pitch axis **PA** relative to the fixed base **110.**

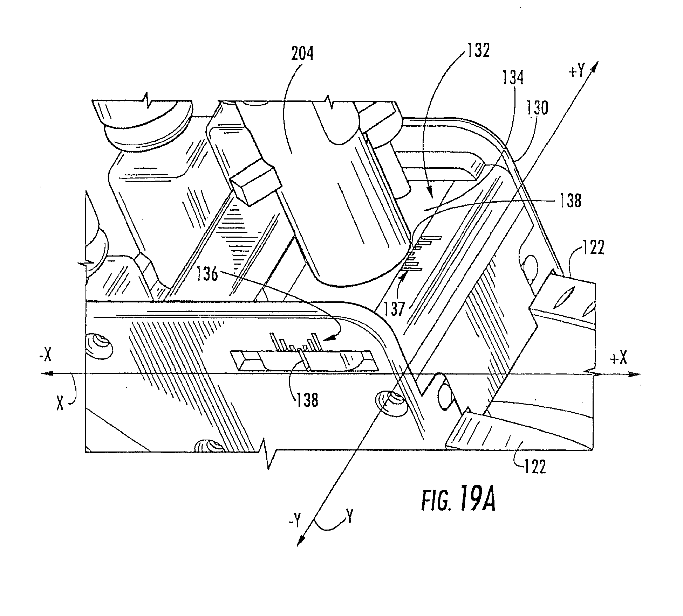

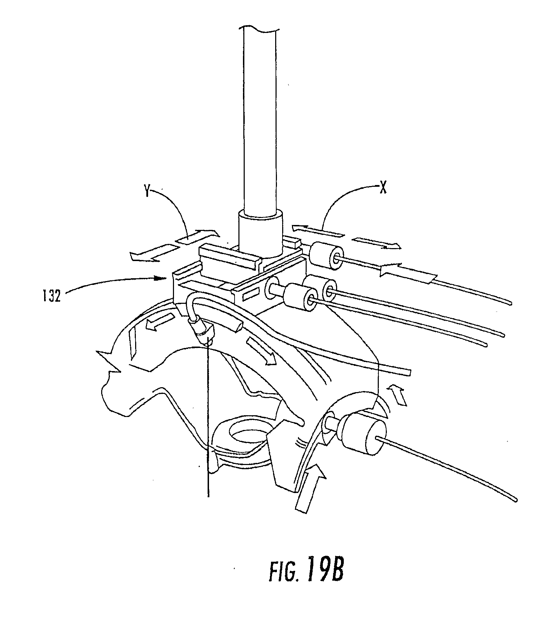

As illustrated in **Fig. 19A****,** the X-Y support table **132** includes a moving plate **134** that moves in both the X-direction and Y-direction. The X-direction actuator **140c,** when rotated, causes translational movement of the moving plate **134** along the X-axis. For example, clockwise rotation of the X-direction actuator **140c** causes movement toward the "-X direction (i.e., to the left) in **Fig. 19A****;** and counterclockwise rotation of the X-direction actuator **140c** causes movement along the +X direction (i.e., to the right) in **Fig. 19A****,** etc. The Y-direction actuator **140d,** when rotated, causes translational movement of the moving plate **134** along the Y-axis. For example, clockwise rotation of the Y-direction actuator **140d** causes movement along the -Y direction (i.e., out of the paper) in **Fig. 19A****;** and clockwise rotation of the Y-direction actuator **140d** causes movement along the +Y direction (i.e., into the paper) in **Fig. 19A****.** In the illustrated embodiment, graduation scales **136, 137** are provided on the platform adjacent the moving plate **134.** The moving plate **134** includes a pair of marks or indicators **138** that provide visual indication of X-Y movement of the moving plate **134.** **Fig. 19B** illustrates X-Y translation of an X-Y support table **132,** according to some embodiments of the present invention.

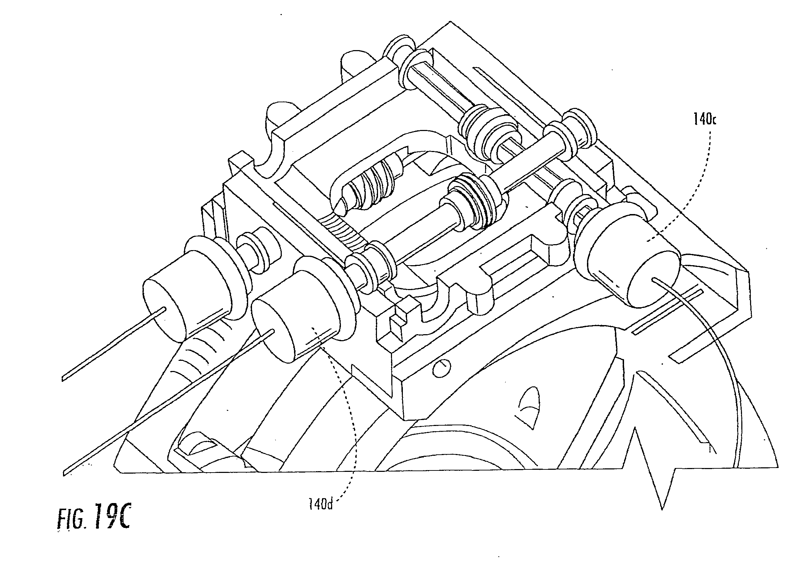

Various internal drive mechanisms may be utilized for causing translational movement of the moving plate **134** in response to user rotation of the X-direction actuator **140c** and the Y-direction actuator **140d.** For example, drive belts, linkages, gears, or worm drives may be utilized, as would be understood by one skilled in the art of X-Y tables. Embodiments of the present invention are not limited to any particular mechanism for translating the X-Y table **132** along the X and Y directions. **Fig. 19C** is partial perspective view of an X-Y support table, according to some embodiments, with elements removed to reveal internal components of an X-direction actuator **140c** and Y-direction actuator **140d.**

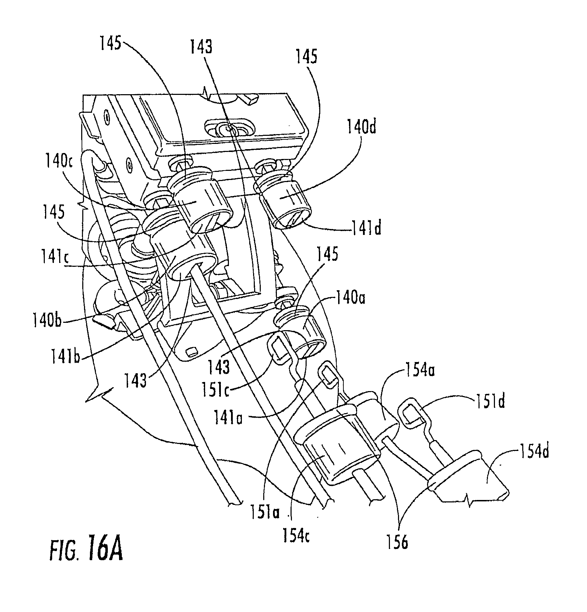

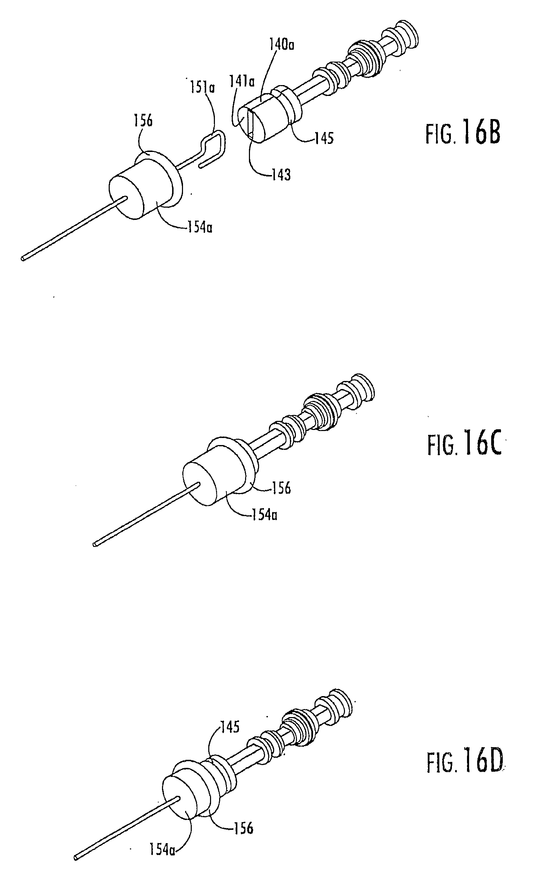

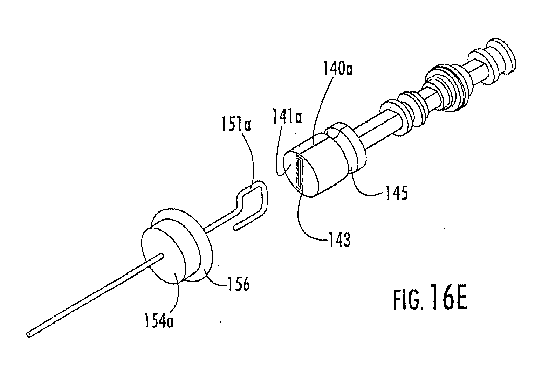

As illustrated in **Fig. 3A****,** the roll actuator **140a,** pitch actuator **140b,** X-direction actuator **140c,** and Y-direction actuator **140d** each extend outwardly from the frame **100** along the same direction (e.g., upwardly from the platform **130).** This configuration facilitates easy connection of the control cables **150a-150d** to the actuators **140a-140d** (where used) and also facilitates bundling of the cables **150a-150d** to reduce clutter or provide ease of handling and set-up. Embodiments of the present invention are not limited to the illustrated embodiment, however. The actuators **140a-140d** may extend in various directions and these directions may be different from each other. In addition, the actuators **140a-140d** may extend along the same direction from the frame, but in a different direction than that illustrated in **Fig. 3A****.** For example, **Fig. 16** illustrates an embodiment where the actuators **140a-140d** extend from a common side of the platform **130.**

Referring to **Figs. 9** and **10A****-10C,** the remote control unit **400** of the illustrated system **50** includes a plurality of manually-operable position controls **402a-402d.** Specifically, the control unit **400** includes a roll adjustment control **402a,** a pitch adjustment control **402b,** an X-direction adjustment control **402c,** and a Y-direction adjustment control **402d.** A roll control cable **150a** is operably connected to the roll adjustment control **402a** and to the roll actuator **140a** such that movement of the roll adjustment control **402a** operates the roll actuator **140a** via the roll control cable **150a.** A pitch control cable **150b** is operably connected to the pitch adjustment control **402b** and to the pitch actuator **140b** such that movement of the pitch adjustment control **402b** operates the pitch actuator **140b** via the pitch control cable **150b.** An X-direction control cable **150c** is operably connected to the X-direction control **402c** and to the X-direction actuator **140c** such that movement of the X-direction adjustment control **402c** operates the X-direction actuator **140c** via the X-direction control cable **150c.** A Y-direction control cable **150d** is operably connected to the Y-direction control **402d** and to the Y-direction actuator **140d** such that movement of the Y-direction adjustment control **402d** operates the Y-direction actuator **140d** via the Y-direction control cable **150d.**

In the illustrated embodiment, each of the position controls **402a-402d** is a thumbwheel control that can be rotated by a user's finger in clockwise and counterclockwise directions. Rotation of each thumbwheel **402a-402d** by a user causes corresponding axial rotation of a respective control cable **150a-150d** and corresponding axial rotation of a respective actuator **140a-140d.**

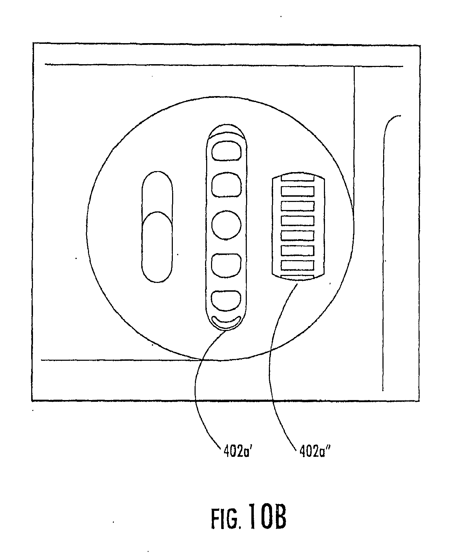

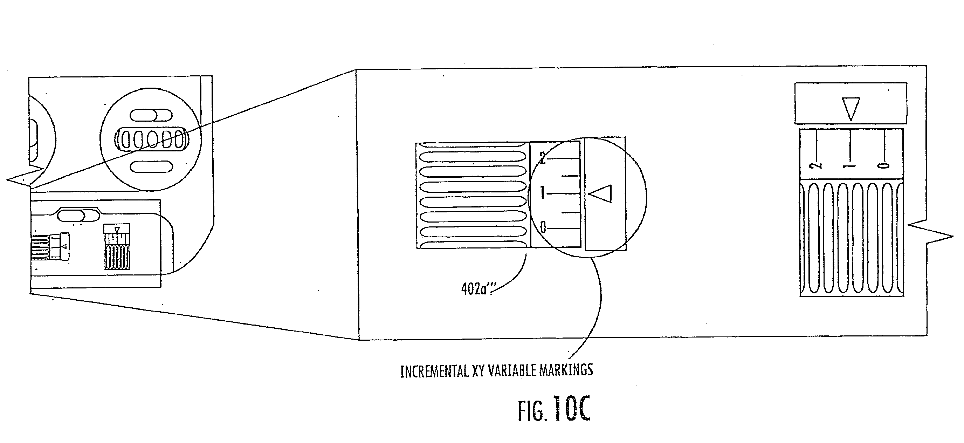

**Fig. 10B** illustrates position controls, according to additional embodiments of the present invention, that utilize two thumbwheels. One thumbwheel **402a'** is for "fine" adjustments; the other thumbwheel **402a"** is for "gross" adjustments. The amount of fine and gross adjustment is correlated to the diameter of each thumbwheel, as would be understood by one skilled in the art. **Fig. 10C** illustrates a position control **402a"',** according to additional embodiments of the present invention, that indicates incremental X-Y variable markings.

In the illustrated embodiment, locking mechanisms **404a-404c** are associated with the thumbwheels **402a-402d** and prevent user rotation thereof when in a locked position. For example, a locking mechanism **404a** is operably associated with the roll adjustment control **402a** and is configured to prevent rotation thereof by a user when in a "locked" position. Locking mechanism **404b** is operably associated with pitch adjustment control **402b** and is configured to prevent rotation thereof by a user when in a "locked" position. Locking mechanism **404c** is operably associated with X-direction control **402c** and **Y-**direction control **402d** and is configured to prevent rotation of X-direction control **402c** and Y-direction control **402d** by a user when in a "locked" position.

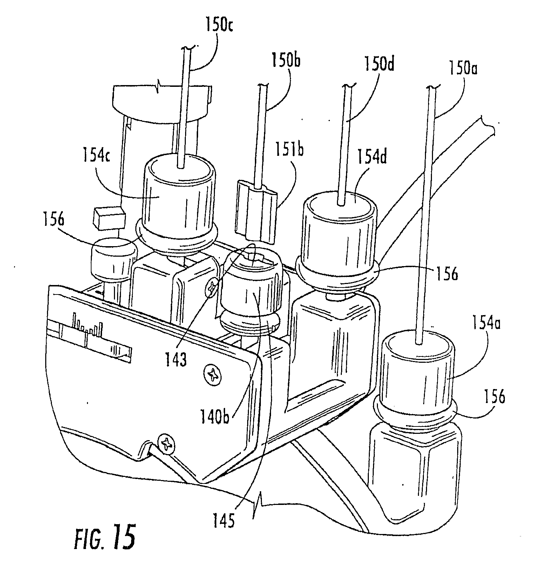

Each control cable **150a-150d** can have a geometrically shaped rigid end **151a-151d** that is configured to removably engage a free end of a respective actuator **140a-140d.** As illustrated in **Fig. 16****,** the respective free ends **141a-141d** of the actuators **140a-140d** may have a slot **143** formed therein that is configured to removably receive a respective cable end. Exemplary cable end shapes include, but are not limited to, "L" shapes, "U" shapes, square shapes, rectangular shapes, oval/circular shapes, and other polygonal shapes. Each cable end has sufficient rigidity such that axial rotation of the cable causes the cable free end to impart rotational motion to a respective actuator. **Fig. 15** illustrates the free end of cable **150b** having a connector **151 b** with a geometric shape attached thereto that is configured to matingly engage a respective slot **143** in actuator **140b,** according to other embodiments of the present invention.

In some embodiments, the free end of an actuator **140a-140d** may be configured to receive only a specific one of the control cables **150a-150d.** For example, in **Fig. 15****,** the connector **151 b** may not fit within the slots **143** of any of the other actuators. As such, a control cable cannot be inadvertently connected to the wrong actuator. For example, the roll adjustment actuator free end **141a** may be configured to only receive the free end **151a** of the control cable **150a** associated with the roll control **402a.** Similarly, the pitch adjustment actuator free end **141b** may be configured to only receive the free end **151b** of the control cable **150b** associated with the pitch control **402b.**

Each control cable **150a-150d** also has a flexible elastomeric (e.g., silicone, rubber, etc.) collar **154a-154d** that is configured to surround a respective actuator **140a-140d** and maintain engagement of the respective cable end **151a-151d** within the respective actuator. Each elastomeric collar **154a-154d** is configured to prevent removal of a cable by a user, for example, as a result of inadvertent tugging on the cable by a user, or by movement of the remote control unit **400.** Each of the illustrated collars **154a-154d** can be rolled or folded back then released to cover and conformably compress against an actuator to hold the end of a respective cable in position. Each collar **154a-154d** can then be pushed back to easily release the cable from the actuator. In the illustrated embodiment, each actuator **140a-140d** has a circumferential groove **145** configured to receive a corresponding circumferential ridge **156** of a collar **154a-154d** in mating relation therewith.

**Fig. 16B** illustrates remote control cable end **151a** about to be inserted into slot **143** in the actuator free end **141a.** The cable end **151a** is inserted into the slot **143** and then the elastomeric collar **154a** is fitted around the actuator **140a** such that the circumferential ridge **156** engages the circumferential actuator groove **145,** as illustrated in **Fig. 16C****.** Because of the elastomeric nature of the collar **154a,** the collar snuggly fits the actuator **140a** and retains the cable end **151a** within slot **143.** To remove the cable end **151a,** the circumferential ridge **156** is pulled out of the groove **145** and the collar **154a** is rolled back on itself as illustrated in **Figs. 16D-16E****.**

Embodiments of the present invention are not limited to the illustrated elastomeric collars **154a-154d.** Other ways of retaining the cable ends **151a-151d** within respective actuators **140a-140d** may be utilized without limitation.

In some embodiments, the actuators **140a-140d** are color coded such that each actuator has a different respective color for easy identification by a user. For example, the roll actuator **140a** may be colored red and the pitch actuator **140b** may be colored yellow such that a user can easily identify the two respective actuators when positioning the frame **100.** In some embodiments, the elastomeric collars **154a-154d** may also be color coded so as to match the color of a respective actuator **140a-140d.** In some embodiments, the cable ends **151a-151d** may also be color coded so as to match the color of a respective actuator **140a-140d.**

In some embodiments of the present invention, the control cables **150a-150d** are formed of NITINOL or other MR-compatible materials. One or more portions of the frame **100** and the targeting cannula **200** may also be formed of NITINOL or other MR-compatible (non-paramagnetic) materials.

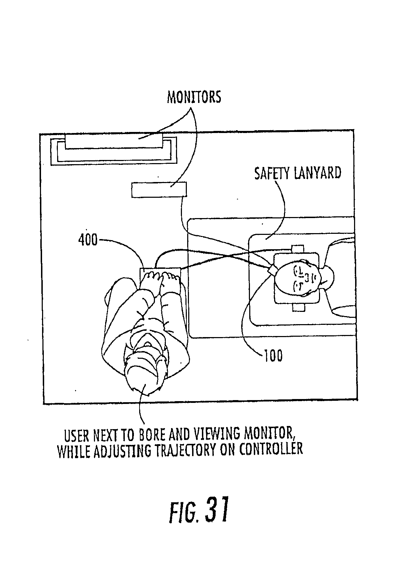

**Fig. 31** illustrates a patient positioned within an MRI scanner and a user utilizing the remote control apparatus **400** and display monitors to adjust the trajectory a targeting cannula.

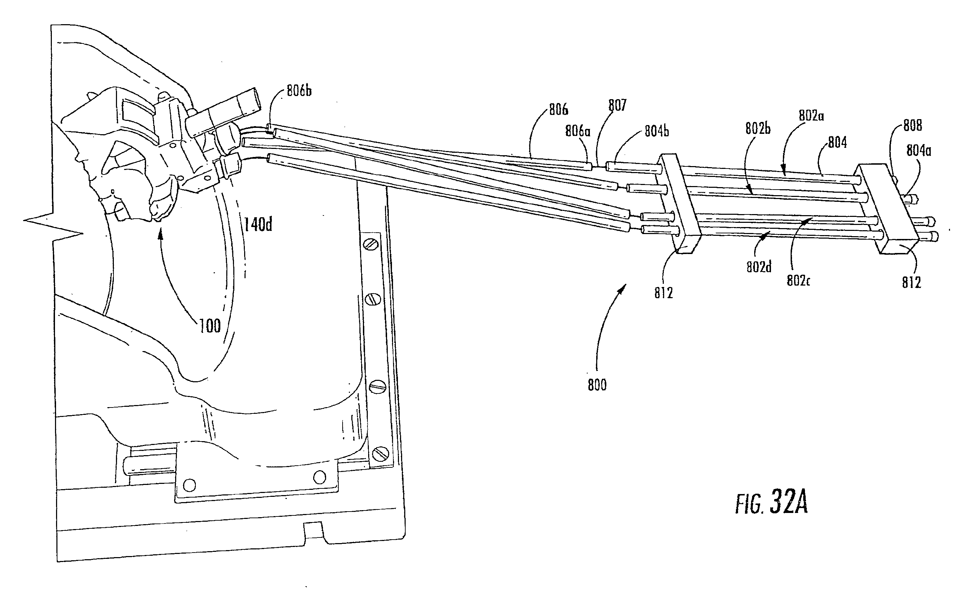

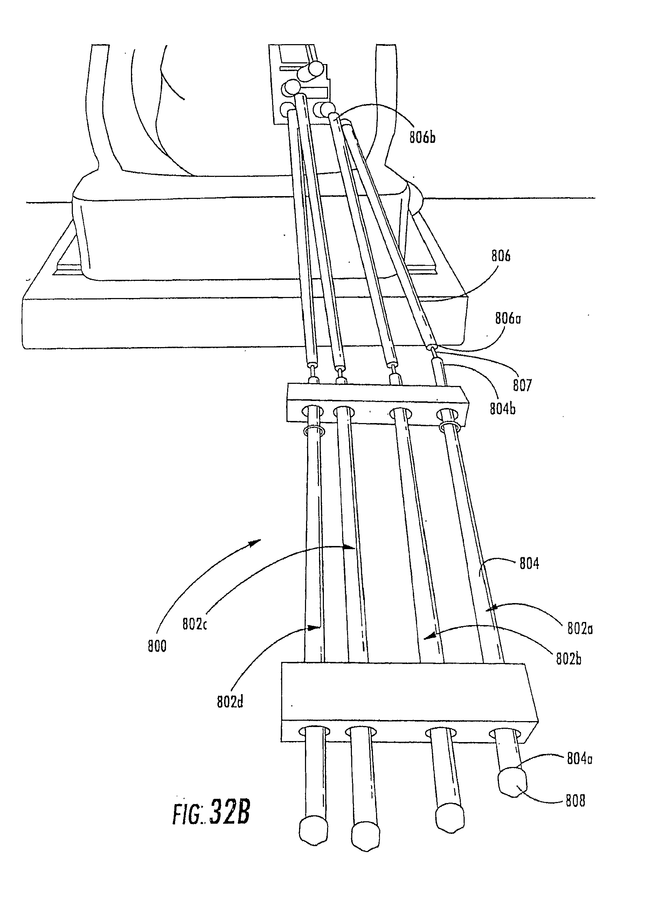

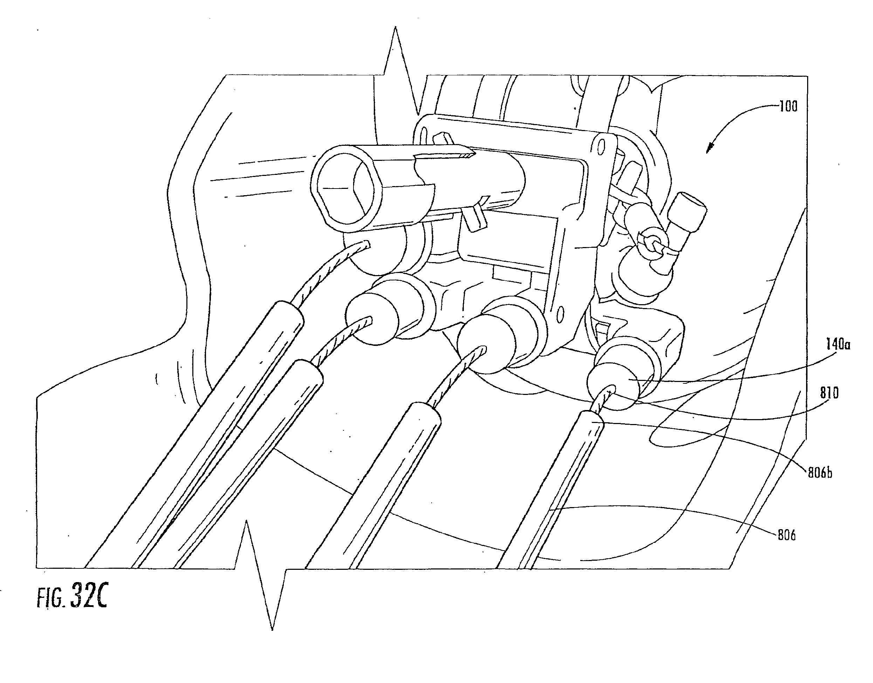

**Figs. 32A-32C** illustrate a remote control unit **800** for remotely controlling the positioning actuators of the frame of **Fig. 3A****,** according to other embodiments of the present invention. The illustrated remote control unit **800** includes a separate, multi-rod control device **802a-802d** for each respective actuator **140a-140d.** Each of the control devices **802a-802d** can be identical in structure and, as such, only one will be described in detail hereinafter. However, each control device **802a-802d** may be color coded with a color different from the other control devices **802a-802d,** and each control device **802a-802d** may have different lengths, shapes or sizes for ease of assembly and/or operation. Moreover, each control device **802a-802d** may be color coded so as to match the color of a respective actuator **140a-140d.**

Each control device **802a-802d** includes a pair of elongated rods **804, 806** (e.g., solid, MRI-compatible rods) joined at respective ends via a flexible member such as a cable **807,** as illustrated. The rods **804, 806** may be formed of wood, polymeric material, or other suitable relatively lightweight MRI-compatible material. In addition, the rods **804, 806** are not required to be solid.

The cable **807** is relatively short in length relative to the length of rods **804, 806** and serves as a universal joint to allow the two rods **804, 806** to rotate even when oriented transverse to each other. For example, the cable **807** may be between about one quarter inch and about one inch (0.25" - 1") in length. However, embodiments of the present invention are not limited to this range for the length of cable **807;** cable **807** may have other lengths. In some embodiments, the cable **807** is an MRI-compatible cable (e.g., NITINOL, etc.). In the illustrated embodiment, the distal end **804b** of rod **804** is joined to the proximal end **806a** of rod **806** via cable **807.** The cable **807** may be joined to rods **804, 806** in any of various ways including, but not limited to, via adhesives, via fasteners, via threaded connections, etc.

The proximal end **804a** of rod **804** includes an endcap **808** secured thereto. The endcap **808** may be formed from tactile material to facilitate rotation of rod **804** by a user. A knob or other device that facilitates rotation may be secured to the proximal end of rod **804** in lieu of endcap **808,** in other embodiments. In operation, a user rotates the proximal end **804a** of rod **804** in a clockwise or counterclockwise direction to correspondingly rotate actuator **140a.**

The distal end **806b** of rod **806** is joined to actuator 140a via a flexible member such as a cable **810,** as illustrated. The cable **810** is relatively short in length relative to the length of rod **806** and serves as a universal joint to allow rod **806** to rotate even when oriented transverse to actuator **140a.** In some embodiments, the cable **810** is an MRI-compatible cable. For example, the cable **810** may be between about one half inch and about one and one half inch (0.5" - 1.5") in length. However, embodiments of the present invention are not limited to this range for the length of cable **810;** cable **810** may have other lengths.

Cable **810** may be joined to rod **806** in any of various ways including, but not limited to, via adhesives, via fasteners, via threaded connections, etc. The free end of cable **810** may have a rigid, geometrical shape, as described above with respect to the embodiments of cables **150a-150d,** and may be configured to engage a slot within the actuator **140a,** as described above. An elastomeric collar, as described above with respect to **Figs. 16A-16E****,** may or may not be necessary to retain the free end of cable **810** within actuator **140a.** In the illustrated embodiment, an elastomeric collar is not utilized. When used, an elastomeric collar can also be color coded to the actuator and/or control device rods **804, 806.**

In the illustrated embodiment, the control devices **802a-802d** are supported by a pair of spaced apart separator devices **812.** Each separator device **812** includes a plurality of substantially parallel, spaced apart bores passing therethrough that are configured to receive each of the rods **804** for the respective control devices **802a-802d.** The separator devices **812** are configured to maintain the rods **804** in substantially parallel, spaced apart relationship, as illustrated. In the illustrated embodiment, only rods **804** pass through the two separator devices **812.** However, embodiments of the present invention are not limited to the illustrated use, configuration, location, or number of separation devices **812.** In addition, although shown as two rods forming each control device **802a-802d,** they may include three or more spaced apart rods (not shown), or only a single rod (not shown). Moreover, other types of devices may be utilized without limitation.

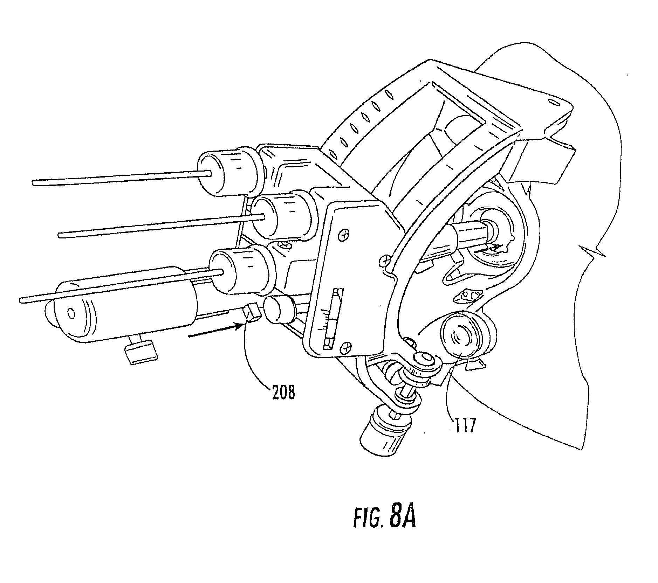

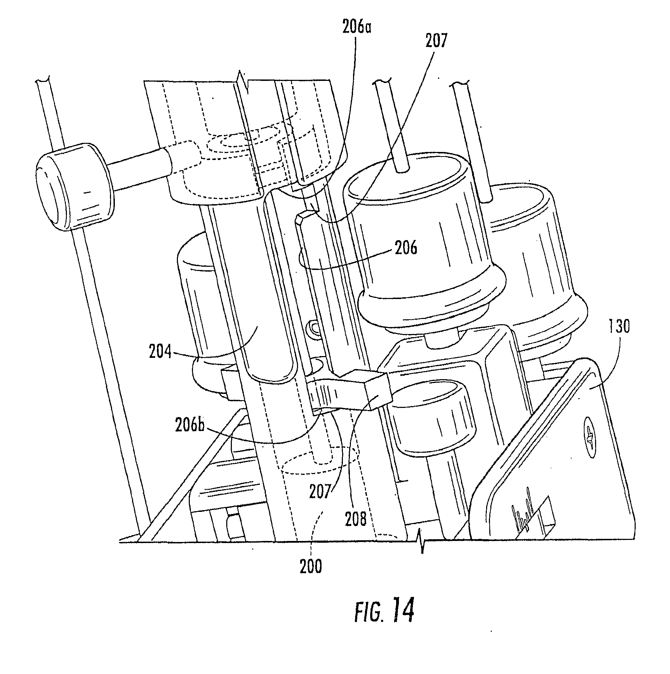

Referring to **Figs. 14** and **19****,** an elongated tubular member **204** extends through the platform **130** and yoke **120** and is secured to the X-Y support table **132.** The targeting cannula **200** is slidably secured within the tubular member **204** and is movable between extended and retracted positions. **Figs. 3A-3D** and **4-6** illustrate the targeting cannula **200** in a retracted position above the burr hole **10** and **Figs. 3E** and **8A** illustrate the targeting cannula 200 in an extended position. The tubular member **204** is configured to lock the targeting cannula **200** in an extended position and in a retracted position, as illustrated in **Fig. 14****.** The tubular member **204** has a pair of radially opposed elongated, axial-extending slots **206,** as illustrated. The ends **206a, 206b** of each slot **206** include a transverse portion **207** that is configured to retain the targeting cannula **200** in respective extended and retracted positions. The targeting cannula **200** includes a respective pair of radially extending prongs **208,** as illustrated. Each prong **208** cooperates with a respective slot **206.** When the targeting cannula **200** is moved upwardly to the retracted position, the targeting cannula **200** is rotated slightly (or the tubular member **204** is rotated slightly) such that prongs **208** each engage a respective transverse portion **207.** When so engaged, the targeting cannula **200** is retained in the retracted position. When the targeting cannula **200** is moved downwardly to the extended position, the targeting cannula **200** is rotated slightly (or the tubular member **204** is rotated slightly) such that prongs **208** each engage a respective transverse portion **207.** When so engaged, the targeting cannula **200** is retained in the extended position.

Embodiments of the present invention are not limited to the illustrated configuration or number of slots **206** in the tubular member **204** or to the number of prongs **208** extending from the targeting cannula **200.** For example, the targeting cannula **200** may have a single prong **208** that is configured to cooperate with a respective single slot **206** in the tubular member **204.** In addition, slot **206** may have a different configuration than illustrated.

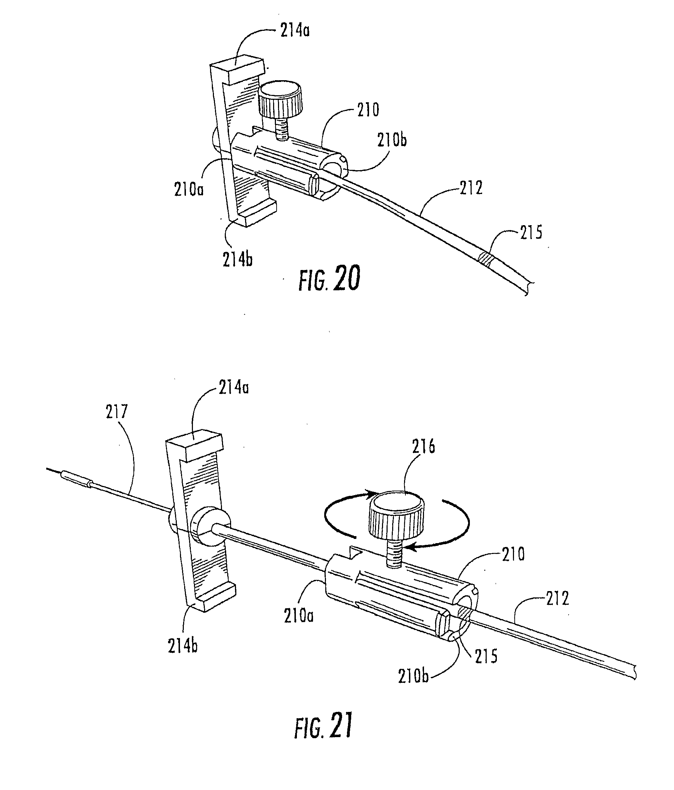

Referring to **Figs. 20-26****,** a depth stop **210** with a removable sheath **212** inserted and secured therein is illustrated. The illustrated depth stop **210** has a generally cylindrical configuration with opposite proximal and distal ends **210a, 210b** and is adapted to be removably secured within the proximal end **204a** of the tubular member **204.** The depth stop **210** is configured to limit a distance that the sheath **212** extends into the body of a patient when the depth stop is inserted within the tubular member **204.** The sheath **212** is configured to receive and guide an elongated interventional device therethrough, as will be described below. The sheath **212** includes opposing tabs **214a, 214b** that, when pulled apart, cause the sheath **212** to peel away for removal from the targeting cannula **200.**

Prior to insertion within the tubular member **204,** the distal end **210b** of the depth stop **210** is positioned adjacent to a mark **215** on the removable sheath **212,** as illustrated in **Fig. 21****.** Locking screw **216** is then tightened to prevent axial movement of the sheath **212** relative to the depth stop **210.** An elongate location verification probe **217,** such as an imaging probe, is then inserted within the sheath **212 as** illustrated in **Fig. 21****.**

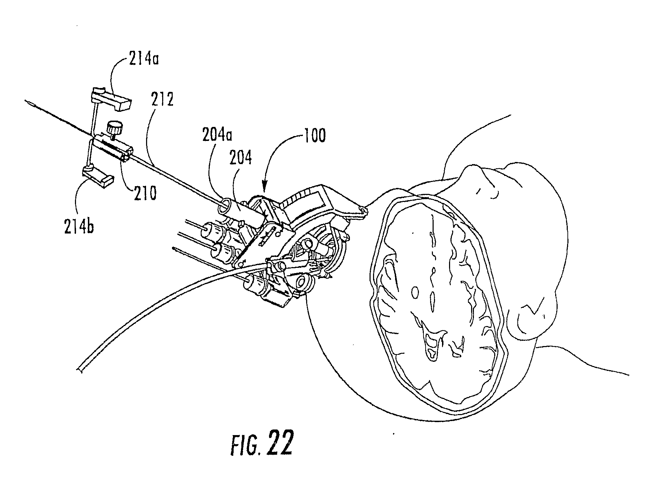

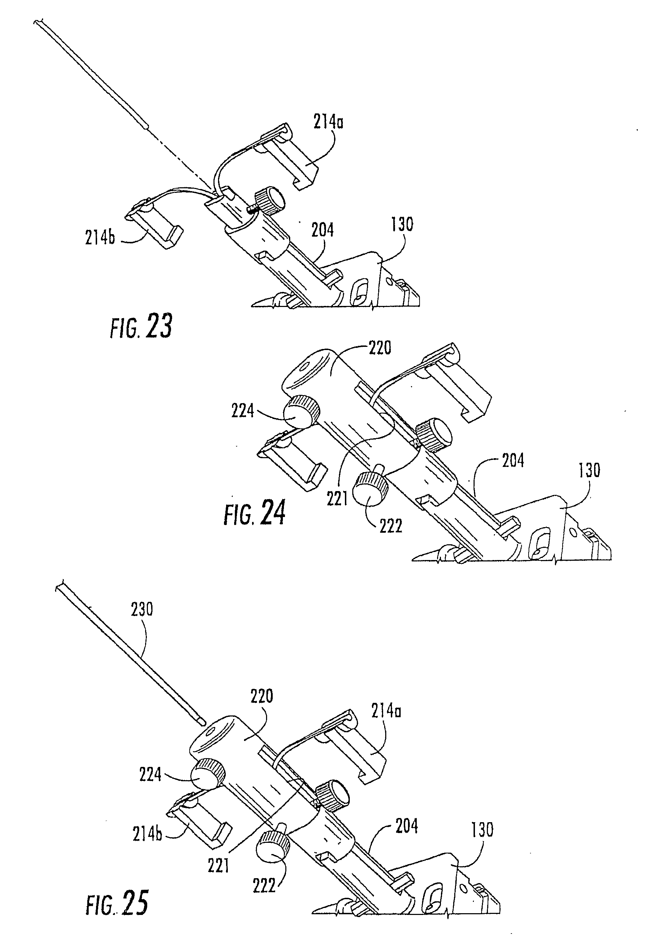

Referring to **Figs. 22-23****,** the opposing tabs **214a, 214b** of the sheath are pulled apart slightly, and the depth stop **210,** sheath **212** and imaging probe 217 are inserted into the proximal end **204a** of tubular member **204.** When so inserted, as illustrated in **Fig. 23****,** the sheath **212** and imaging probe **217** pass through the axial bore of the targeting cannula **200.** The imaging probe **217** is then utilized to verify that the distal end **212b** of the sheath 212 is positioned at the correct location within the body of a patient. Upon verifying that the sheath **212** is accurately positioned, the imaging probe **217** is removed **(****Fig. 23****).**

The probe **217** extending through the targeting cannula guide bore can include at least one electrode on a distal tip portion thereof. The electrode can be a recording and/or stimulating electrode. The electrode can be configured to deliver test voltages for physiologic confirmation of location/efficacy that can be done by fMRI or by feedback from a non-anesthetized patient. Thus, a patient can be stimulated with an interventional probe (the stimulation may be via a transducer on a distal tip portion of the probe), to help confirm that the interventional probe is in the correct location (i.e., confirm proper location via anatomical as well as provide physiologic information and feedback). During (and typically substantially immediately after) stimulation from the interventional probe, the physician can monitor for a physiologic response from the patient that can be observed either directly from the patient as a physical response or via an fMRI-visible response.

The elongate probe **217** can be MRI-visible and may optionally be configured to define an MRI antenna. The system **50** can be configured to allow for real-time tracking under MRI, with an SNR imaging improvement in a diameter of at least 5-10 mm proximate the probe or targeting cannula **200.**

Next, a locking mechanism **220** is removably secured to the proximal end **210a** of the depth stop **210.** The locking mechanism **202** includes opposing axially extending slots **221,** as illustrated. The portions of the sheath **212** that have been peeled away extend through these slots as illustrated. The locking mechanism **220** is secured to the depth stop/tubular member via locking screw **222.**

The targeting cannula 200 is now ready to receive an interventional device therein. As illustrated in **Fig. 25****,** an interventional device **230,** such as a brain stimulation lead, is inserted into the locking mechanism and through the removable sheath **212.** A locking screw **224** is tightened to secure the interventional device **230** and prevent axial movement thereof.

The targeting cannula **200** can be MRI-visible. In some particular embodiments, the cannula **200** may optionally comprise a plurality of spaced apart microcoils configured to provide data used to provide 3-D dimensional data in MRI 3-D space, such as a trajectory, or 3-D spatial coordinates of position of the cannula **200.** The microcoils can each provide data that can be correlated to a three-dimensional (X,Y, Z) position in 3-D space in the body. The mircocoils can be in communication with the MRI scanner, and tracking sequences can be generated and data from one or more of the MRI scanner channels can be used to define positional 3-D positional data and a trajectory thereof.

In some particular embodiments, the progress of the cannula **200** and/or interventional probe **230** or other device may optionally be tracked in substantially real-time as it advances to the target via the coils (similar ones of which may also or alternatively be on or in the probe or other device) and/or antenna. However, real-time tracking may not be desired in some embodiments.

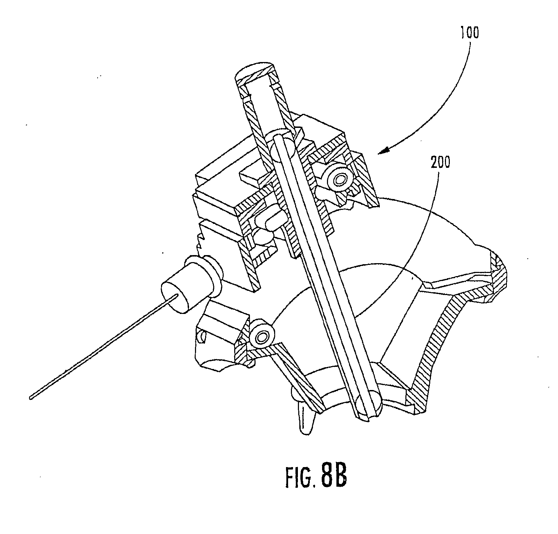

In some embodiments, the cannula **200** can include at least one axially extending fluid-filled hollow lumen **(****Fig. 8B****)** or closed channel with fluid that can generate MRI signal that can be detected by an MRI scanner and/or by an internal MRI antenna incorporated on and/or into the cannula **200** that can increase the SNR of the fluid to increase its visibility in an MRI. The fluid may be an aqueous solution (able to resonate at the proton frequency). The cannula **200** can include an axially extending, relatively thin segment, which creates a high contrast MRI image (a segment filled with water or other suitable contrast solution filled section/lumen). The thickness of the segment may be between about 0.25-4 mm (and the segment can have a tubular shape with a diameter or may define another cross-sectional shape such as a square section). The cannula **200** may include MRI imaging coils to increase the signal from the high contrast fluid. See, e.g., co-pending U.S. Patent Application Serial No. 12/066,862.

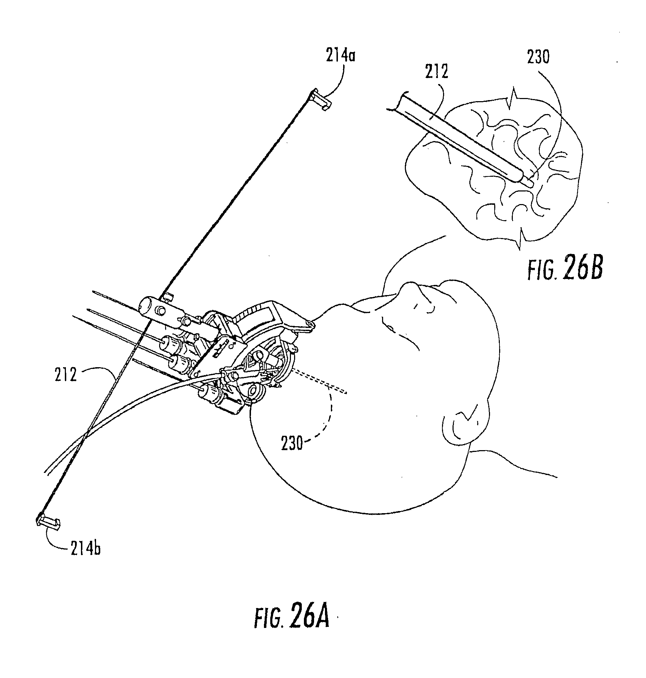

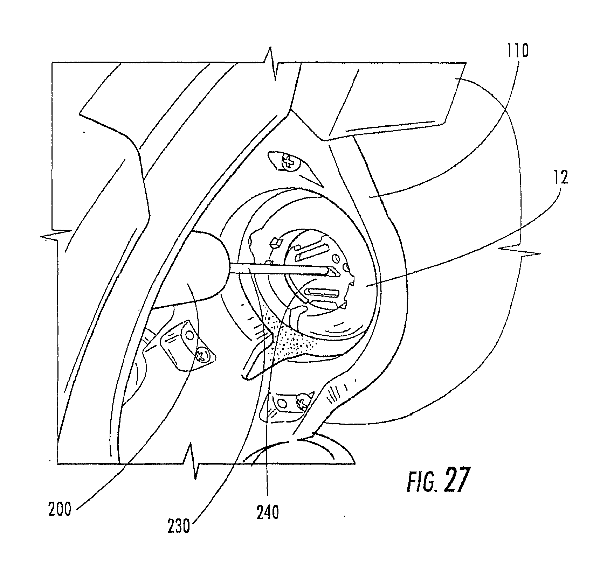

As illustrated in **Figs. 26A** and **26B****,** the interventional device **230** is positioned at the desired location within the body of a patient. The removable sheath **212** is then completely removed by pulling apart the opposing tabs **214a, 214b.** A clamp **240 (****Fig. 27****)** is inserted within the burr hole ring **12** that grips the interventional device **230** to prevent inadvertent removal of the interventional device **230** from the body of the patient. The clamp **240** is secured to the burr hole ring **12.** The frame **100** can then be removed from the body of the patient, leaving behind only the interventional device **230** that has been inserted into the patient body.

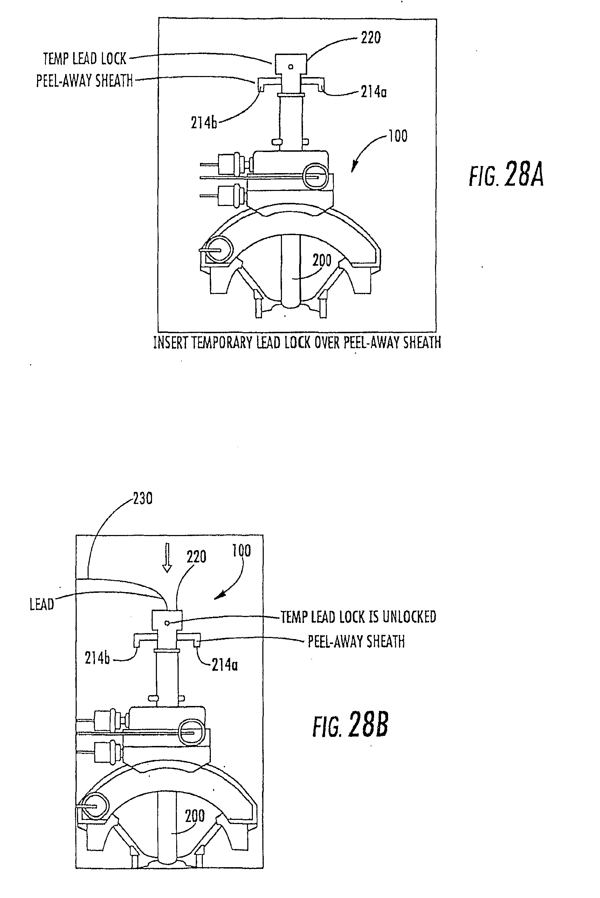

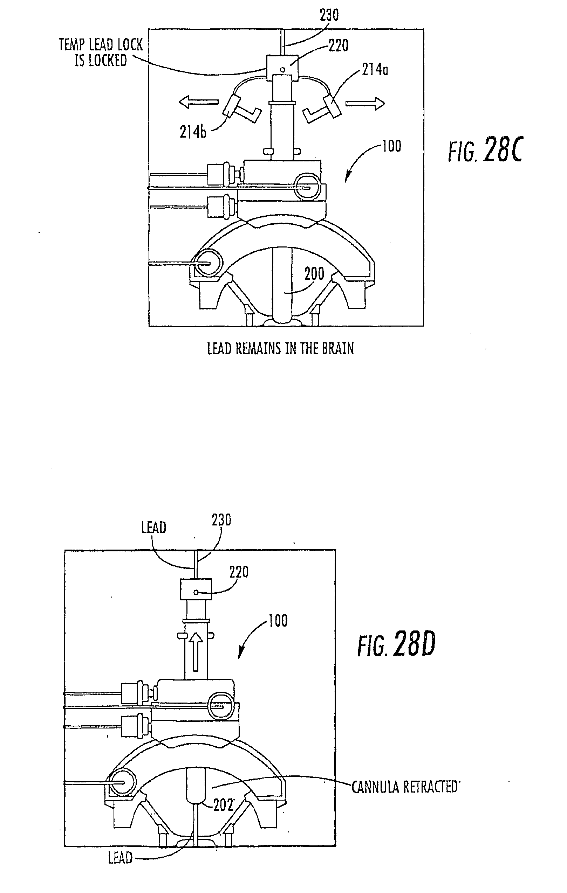

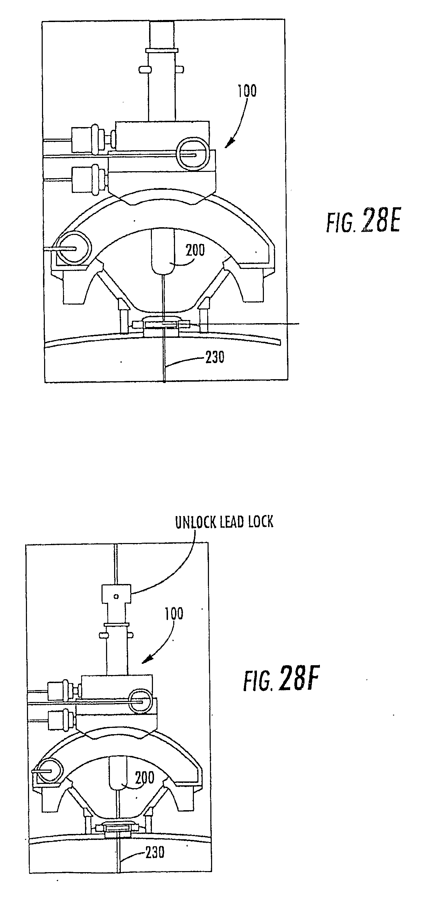

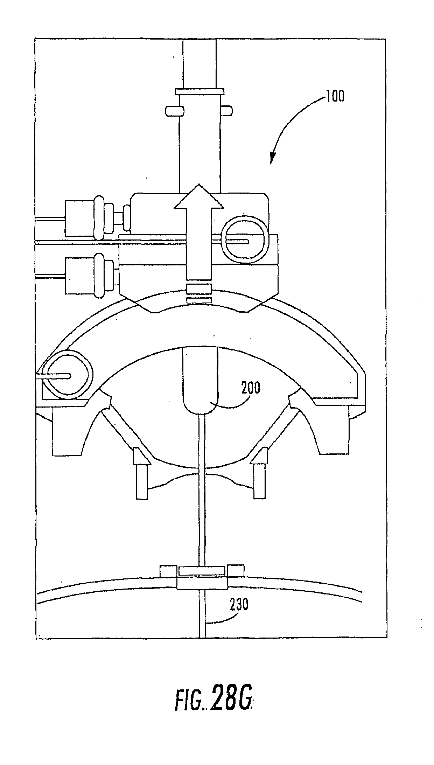

**Figs. 28A-28G** are side view, schematic illustrations of the trajectory frame **100** illustrating an exemplary series of operations for the insertion of interventional devices within the body of a patient via the targeting cannula **200.** In **Fig. 28A****,** the locking mechanism **220,** depth stop **210,** and removable sheath **212** are positioned within the targeting cannula **200.** In **Fig. 28B****,** an interventional device, e.g., lead **230,** is inserted within the sheath **212** and into the brain of the patient. The locking mechanism **220** secures the lead against axial movement **(****Fig. 28C****).** The targeting cannula **200** is then retracted **(****Figs. 28D-28E****).** The locking mechanism **220** is unlocked **(****Fig. 28F****)** and the frame **100** is removed from the skull of the patient **(****Fig. 28G****).**

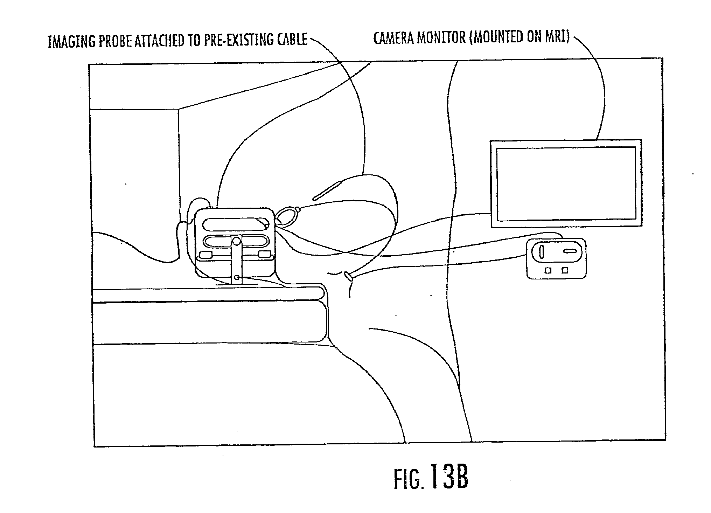

In some embodiments of the present invention, a video imaging device (e.g., a fiber optic probe with visual access to the burr hole **10** and/or trajectory frame **100** in communication with a camera and display in a clinician workstation) **500** for viewing the burr hole **10** is removably secured to the frame **100** or to the targeting cannula tubular member **204** via a bracket **502.** For example, as illustrated in **Fig. 13A****,** a bracket **502** is secured to the targeting cannula tubular member **204.** The illustrated bracket **502** is configured to adjustably slide axially along the tubular member **204** for positioning. The illustrated bracket **502** includes a sleeve **504** that is configured to slidably receive an imaging device 500 therein. Threaded locking device 506 is configured to secure the imaging device 500 in position within the sleeve 504 for positioning of the imaging device 500 relative to the body of a patient. A clinician views images captured by the video imaging device 500 via a monitor, as illustrated in Fig. **13B****.**

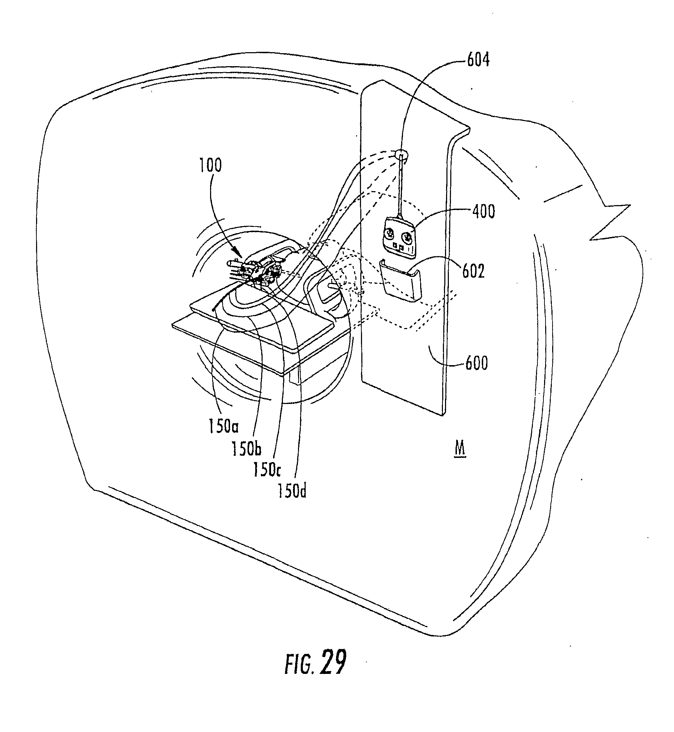

In the illustrated embodiment of Fig. 29, a sterile drape 600 is provided for holding the remote control unit 400. The drape 600 is configured to be positioned near the body of a patient and includes a pocket 602 configured to removably receive the remote control unit 400 therein. The drape 600 also includes an aperture 604 through which the cables 150a-150b extend from the remote control unit **400** to the frame **100.** In the illustrated embodiment, the drape **600** is attached to the magnet housing **M** of an MRI scanner system.

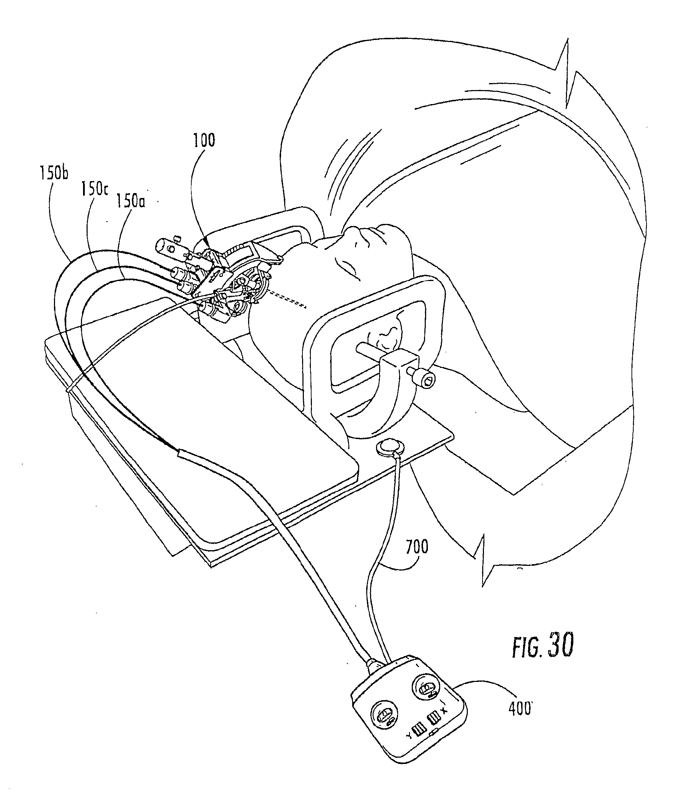

In some embodiments, the control cables **150a-150d** are configured to have a limited length. Accordingly, as illustrated in **Fig. 30****,** a safety lanyard **700** may be attached to the remote control unit **400** and to a rigid object to prevent inadvertent detachment of the control cables **150a-150d** from the frame actuators **140a-140d** caused by moving the remote control unit **400** too far from the frame **100.**

Operations associated with a typical surgical procedure using the trajectory frame **100**will now be described. These operations relate to deep brain stimulation procedures. Embodiments of the present invention are not limited to use with deep brain stimulation procedures, however.

Initially, a patient is placed within an MR scanner and MR images are obtained of the patient's head that visualize the patient's skull, brain, fiducial markers and ROI (region of interest or target therapeutic site). The MR images can include volumetric high-resolution images of the brain. To identify the target ROI, certain known anatomical landmarks can be used, i.e., reference to the AC, PC and MCP points (brain atlases give the location of different anatomies in the brain with respect to these point) and other anatomical landmarks. The location of the burr hole **10** may optionally be determined manually by placing fiducial markers on the surface of the head or programmatically by projecting the location in an image.

Images in the planned plane of trajectory are obtained to confirm that the trajectory is viable, i.e., that no complications with anatomically sensitive areas should occur. The patient's skull is optically or manually marked in one or more desired locations to drill the burr hole. The burr hole **10** is drilled and a burr hole ring **12** is affixed to the skull overlying the burr hole.

The trajectory frame **100** is then fixed to the skull of the patient and the targeting cannula **200** is properly fitted thereto. A localization scan is obtained to determine/register the location of the targeting cannula **200,** in direct orientation of the trajectory frame **100.** The settings to which the trajectory frame **100** should be adjusted are electronically determined so that the targeting cannula **200** is in the desired trajectory plane. Frame adjustment calculations are provided to a clinician who can manually or electronically adjust the orientation of the frame **100.** The desired trajectory plane is confirmed by imaging in one or more planes orthogonal to the desired trajectory plane.

Once the targeting cannula **200** has the desired trajectory plane, the multipurpose probe **217** and delivery sheath **212** are advanced through the targeting cannula **200.** The advancement of the probe **217** is monitored by imaging to verify that the probe will reach the target accurately. If the probe **217** and delivery sheath **212** are at the desired target, the sheath is left in place and the probe **217** is removed. The sheath **212** will now act as the delivery cannula for the implantable lead **230.**

If the probe **217** and delivery sheath **212** are not at the desired/optimal location, a decision is made as to where the probe **217** and delivery sheath **212** need to be. The trajectory frame **100** is adjusted accordingly via the actuators **140a-140d** and the probe **217** and delivery sheath **212** are re-advanced into the brain. Once the probe **217** and delivery sheath **212** are at the desired location, the probe **217** is removed and the delivery sheath is left in place. The lead **230** is then advanced to the target location using the sheath **212** as a guide. The location of the lead is confirmed by reviewing an image, acoustic recording and/or stimulation. The sheath **212** is then removed, leaving the lead in place.

It is contemplated that embodiments of the invention can provide an integrated system **50** that may allow the physician to place the interventional device/leads accurately and in short duration of time. In some embodiments, once the burr hole is drilled, and the trajectory frame is fixed to the skull; the trajectory frame is oriented such that the interventional device advanced using the trajectory frame follows the desired trajectory and reaches the target as planned in preoperative setup imaging plans. As described herein, the system **50** can employ hardware and software components to facilitate an automated or semiautomated operation to carry out this objective.

The invention is defined by the following claims.

## Claims

1. An MRI-guided interventional system (50), comprising: a cooperating targeting cannula (200); a frame (100) that supports the cooperating targeting cannula (200), wherein the frame is configured to translate and rotate such that the targeting cannula can be positioned to a desired intrabody trajectory, and wherein the targeting cannula includes a guide bore therethrough that is configured to guide placement of an interventional device in vivo, wherein the frame comprises:
a base (110) having a patient access aperture (112) formed therein, wherein the base is configured to be secured to the body of a patient;
a yoke (120) movably mounted to the base and rotatable about a roll axis, to thereby rotate the cannula;
a platform (130) movably mounted to the yoke and rotatable about a pitch axis; and an X-Y support table (132), wherein the cannula is secured to the X-Y support table and wherein the X-Y support table (132) is movably mounted to the platform and is configured to translate in both an X-direction and Y-direction relative to the platform, to thereby translate the cannula; and
a plurality of user-activatable actuators (140a - 140d) operably connected to the frame that are configured to translate and rotate the frame relative to the body of the patient so as to position the targeting cannula to a desired intrabody trajectory.

2. The system of Claim 1, wherein the frame comprises:
a roll actuator (140a) operably connected to the yoke and configured to rotate the yoke about the roll axis;
a pitch actuator (140b) operably connected to the platform and configured to rotate the platform about the pitch axis;
an X-direction actuator (140c) operably connected to the platform and configured to move the X-Y support table in the X-direction; and
a Y-direction actuator (140d) operably connected to the platform and configured to move the X-Y support table in the Y-direction.

3. The system of Claim 1, wherein the yoke comprises a pair of spaced apart arcuate arms (122), and wherein the platform engages and moves along the arcuate arms when rotated about the pitch axis.

4. The system of Claim 3, wherein at least one of the yoke arcuate arms comprises a thread pattern (124) formed in a surface thereof, and wherein the pitch actuator comprises a rotatable worm (146) with teeth (148) configured to engage the thread pattern, and wherein rotation of the worm causes the platform to rotate about the pitch axis.

5. The system of Claim 1, wherein the base comprises a pair of spaced apart arcuate arms (116), and wherein the yoke engages and moves along the arcuate arms when rotated about the roll axis.

6. The system of Claim 5, wherein at least one of the base arcuate arms comprises a thread pattern (118) formed in a surface thereof, and wherein the roll actuator comprises a rotatable worm (142) with teeth configured to engage the thread pattern, and wherein rotation of the worm causes the yoke to rotate about the roll axis.

7. The system of Claim 2, wherein the roll actuator, pitch actuator, X-direction actuator, and Y-direction actuator each have respective free ends (141a - 141 d) which extend outwardly from the frame along a common direction.

8. The system of Claim 1, further comprising:
a remote control unit (400, 800) comprising a plurality of controls; and
a plurality of control cables (150a - 150d), each cable operably connected to a respective control and to a respective actuator, wherein movement of a control operates a respective actuator via a respective control cable.

9. The system of Claim 8, wherein the control unit comprises a roll adjustment control (402a), a pitch adjustment control (402b), an X-direction adjustment control (402c), and a Y-direction adjustment control (402d);
wherein a roll control cable (150a) is operably connected to the roll adjustment control and to the roll actuator, wherein movement of the roll adjustment control operates the roll actuator via the roll control cable;
wherein a pitch control cable (150b) is operably connected to the pitch adjustment control and to the pitch actuator, wherein movement of the pitch adjustment control operates the pitch actuator via the pitch control cable;
wherein an X-direction control cable (150c) is operably connected to the X-direction control and to the X-direction actuator, wherein movement of the X-direction adjustment control operates the X-direction actuator via the X-direction control cable; and
wherein a Y-direction control cable (150d) is operably connected to the Y-direction control and to the Y-direction actuator, wherein movement of the Y-direction adjustment control operates the Y-direction actuator via the Y-direction control cable.

10. The system of Claim 8, wherein the control cables are MRI compatible cables.

11. The system of Claim 1, further comprising a video imaging probe (500) removably secured to the frame.

12. The system of Claim 1, further comprising an elongated tubular member (204) extending through the platform and yoke and secured to the X-Y table, wherein the targeting cannula is slidably secured within the tubular member and is movable between extended and retracted positions, and wherein the targeting cannula is configured to translate in response to translational movement of the X-Y support table and to rotate in response to rotational movement of the yoke and platform to define different axial trajectories extending through the patient access aperture.

13. The system of Claim 1, further comprising a plurality of MRI-visible fiducial markers (117) attached to the frame.

## Patentansprüche

1. Ein MRT-geführtes Interventionssystem (50), das Folgendes beinhaltet: eine zusammenwirkende Zielkanüle (200);
einen Rahmen (100), der die zusammenwirkende Zielkanüle (200) stützt, wobei der Rahmen zum Verschieben und Drehen konfiguriert ist, so dass die Zielkanüle auf eine erwünschte Bahn im Körperinnern positioniert werden kann, und wobei die Zielkanüle eine Führungsbohrung durch sie hindurch aufweist, die zum Führen des Setzens einer Eingriffsvorrichtung in vivo konfguriert ist, wobei der Rahmen Folgendes beinhaltet:
eine Basis (110) mit einer in ihr ausgebildeten Patientenzugangsöffnung (112), wobei die Basis zum Befestigen am Körper eines Patienten konfiguriert ist;
ein Joch (120), das beweglich an der Basis montiert ist und um eine Rollachse drehbar ist, um somit die Kanüle zu drehen;
eine Plattform (130), die beweglich an dem Joch montiert ist und um eine Nickachse drehbar ist; und einen X-Y-Auflagetisch (132), wobei die Kanüle an dem X-Y-Auflagetisch befestigt ist und wobei der X-Y-Auflagetisch (132) beweglich an der Plattform montiert ist und zum Verschieben sowohl in einer X-Richtung als auch einer Y-Richtung relativ zu der Plattform konfiguriert ist, um somit die Kanüle zu verschieben; und
eine Vielzahl von anwenderaktivierbaren Stelleinheiten (140a - 140d), die betriebsfähig mit dem Rahmen verbunden sind, die zum Verschieben und Drehen des Rahmens relativ zu dem Körper des Patienten konfiguriert sind, um die Zielkanüle auf einer erwünschten Bahn im Körperinnern zu positionieren.

2. System gemäß Anspruch 1, wobei der Rahmen Folgendes beinhaltet:
eine Rollstelleinheit (140a), die betriebsfähig mit dem Joch verbunden ist und zum Drehen des Jochs um die Rollachse konfiguriert ist;
eine Nickstelleinheit (140b), die betriebsfähig mit der Plattform verbunden ist und zum Drehen der Plattform um die Nickachse konfiguriert ist;
eine X-Richtung-Stelleinheit (140c), die betriebsfähig mit der Plattform verbunden ist und zum Bewegen des X-Y-Auflagetischs in der X-Richtung konfiguriert ist; und
eine Y-Richtung-Stelleinheit (140d), die betriebsfähig mit der Plattform verbunden ist und zum Bewegen des X-Y-Auflagetischs in der Y-Richtung konfiguriert ist.

3. System gemäß Anspruch 1, wobei das Joch ein Paar von beabstandeten bogenförmigen Armen (122) beinhaltet und wobei die Plattform eingreift und sich an den bogenförmigen Armen entlang bewegt, wenn sie um die Nickachse gedreht wird.

4. System gemäß Anspruch 3, wobei mindestens einer der bogenförmigen Arme des Jochs ein Gewindemuster (124) beinhaltet, das in einer Oberfläche davon ausgebildet ist, und wobei die Nickstelleinheit eine drehbare Schnecke (146) mit Zähnen (148) beinhaltet, die zum Eingriff mit dem Gewindemuster konfiguriert sind, und wobei die Drehung der Schnecke dazu führt, dass die Plattform um die Nickachse gedreht.

5. System gemäß Anspruch 1, wobei die Basis ein Paar von beabstandeten bogenförmigen Armen (116) beinhaltet und wobei das Joch eingreift und sich an den bogenförmigen Armen entlang bewegt, wenn es um die Rollachse gedreht wird.

6. System gemäß Anspruch 5, wobei mindestens einer der bogenförmigen Arme der Basis ein Gewindemuster (118) beinhaltet, das in einer Oberfläche davon ausgebildet ist, und wobei die Rollstelleinheit eine drehbare Schnecke (142) mit Zähnen beinhaltet, die zum Eingriff mit dem Gewindemuster konfiguriert ist, und wobei die Drehung der Schnecke dazu führt, dass das Joch um die Rollachse gedreht.

7. System gemäß Anspruch 2, wobei die Rollstelleinheit, Nickstelleinheit, X-Richtung-Stelleinheit und Y-Richtung-Stelleinheit jeweils freie Enden (141a - 141d) aufweisen, die sich einer gemeinsamen Richtung entlang vom Rahmen nach außen erstrecken.

8. System gemäß Anspruch 1, das ferner Folgendes beinhaltet:
eine Fernsteuerungseinheit (400, 800), die eine Vielzahl von Steuerungen beinhaltet; und
eine Vielzahl von Steuerkabeln (150a - 150d), wobei jedes Kabel betriebsfähig mit einer jeweiligen Steuerung und einer jeweiligen Stelleinheit verbunden ist, wobei die Bewegung einer Steuerung eine jeweilige Stelleinheit über ein jeweiliges Steuerkabel betreibt.

9. System gemäß Anspruch 8, wobei die Steuerungseinheit eine Rolljustiersteuerung (402a), eine Nickjustiersteuerung (402b), eine X-Richtung-Justiersteuerung (402c) und eine Y-Richtung-Justiersteuerung (402d) beinhaltet;
wobei ein Rollsteuerkabel (150a) betriebsfähig mit der Rolljustiersteuerung und der Rollstelleinheit verbunden ist, wobei die Bewegung der Rolljustiersteuerung die Rollstelleinheit über das Rollsteuerkabel betreibt;
wobei ein Nicksteuerkabel (150b) betriebsfähig mit der Nickjustiersteuerung und der Nickstelleinheit verbunden ist, wobei die Bewegung der Nickjustiersteuerung die Nickstelleinheit über das Nicksteuerkabel betreibt;
wobei ein X-Richtung-Steuerkabel (150c) betriebsfähig mit der X-Richtung-Steuerung und der X-Richtung-Stelleinheit verbunden ist, wobei die Bewegung der X-Richtung-Justiersteuerung die X-Richtung-Stelleinheit über das X-Richtung-Steuerkabel betreibt; und
wobei ein Y-Richtung-Steuerkabel (150d) betriebsfähig mit der Y-Richtung-Steuerung und der Y-Richtung-Stelleinheit verbunden ist, wobei die Bewegung der Y-Richtung-Justiersteuerung die Y-Richtung-Stelleinheit über das Y-Richtung-Steuerkabel betreibt.

10. System gemäß Anspruch 8, wobei die Steuerkabel MRT-kompatible Kabel sind.

11. System gemäß Anspruch 1, das ferner eine Bildgebungssonde (500) beinhaltet, die entfernbar an dem Rahmen befestigt ist.

12. System gemäß Anspruch 1, das ferner ein längliches röhrenförmiges Element (204) beinhaltet, das sich durch die Plattform und das Joch erstreckt und an dem X-Y-Tisch befestigt ist, wobei die Zielkanüle gleitfähig in dem röhrenförmigen Element befestigt ist und zwischen ausgezogenen und zurückgezogenen Positionen bewegt werden kann, und wobei die Zielkanüle dazu konfiguriert ist, als Reaktion auf eine verschiebende Bewegung des X-Y-Auflagetischs zu verschieben und als Reaktion auf eine Drehbewegung des Jochs und der Plattform zu drehen, um verschiedene Achsenbahnen zu definieren, die sich durch die Patientenzugangsöffnung erstrecken.

13. System gemäß Anspruch 1, das ferner eine Vielzahl von MRT-sichtbaren Lokalisationsmarkern (117) beinhaltet, die an dem Rahmen angebracht sind.

## Revendications

1. Système d'intervention guidé par IRM (50), comprenant : une canule d'acheminement (200) ;
un châssis (100) qui supporte la canule d'acheminement (200),
dans lequel lequel le châssis est conçu pour translater et pivoter de telle sorte que la canule d'acheminement puisse être positionnée dans une trajectoire intracorporelle souhaitée, et dans lequel la canule d'acheminement comprend un alésage de guidage ménagé à l'intérieur de cette canule, lequel alésage est conçu pour guider l'installation in vivo d'un dispositif d'intervention, dans lequel le châssis comprend :
une base (110) qui comprend une ouverture d'accès au patient (112) formée dans celle-ci, la base étant conçue pour être fixée au corps d'un patient ;
une fourche (120) montée de manière mobile à la base et rotative autour d'un axe de roulis, pour ainsi faire tourner la canule ;
une plate-forme (130) montée de manière mobile à la fourche et rotative autour d'un axe de tangage ; et une table de support X-Y (132), dans laquelle la canule est fixée à la table de support X-Y et dans laquelle la table de support X-Y (132) est montée
de manière mobile à la plate-forme et est conçue pour translater à la fois dans les directions X et Y par rapport à la plate-forme, pour ainsi translater la canule ; et
une pluralité d'actionneurs activables par l'utilisateur (140a - 140d) raccordés fonctionnellement au châssis, lesquels sont conçus pour translater et faire pivoter le châssis par rapport au corps du patient de manière à positionner la canule d'acheminement dans une trajectoire intracorporelle souhaitée.

2. Système selon la revendication 1, dans lequel le châssis comprend :
un actionneur de roulis (140a) raccordé fonctionnellement à la fourche et conçu pour faire pivoter la fourche autour de l'axe du roulis ;
un actionneur de tangage (140b) raccordé fonctionnellement à la plate-forme et conçu pour faire pivoter la plate-forme autour de l'axe de tangage ;
un actionneur en direction X (140c) raccordé fonctionnellement à la plate-forme et conçu pour déplacer la table de support X-Y dans la direction X ;
un actionneur en direction Y (140d) raccordé fonctionnellement à la plate-forme et conçu pour déplacer la table de support X-Y dans la direction Y.

3. Système selon la revendication 1, dans lequel la fourche comprend une paire de bras arqués espacés (122) et dans lequel la plate-forme met en prise et se déplace le long des bras arqués lors d'une rotation autour de l'axe de tangage.

4. Système selon la revendication 3, dans lequel au moins un des bras arqués de la fourche comprend un motif de filetage (124) formé dans une surface de celle-ci, et dans lequel l'actionneur de tangage comprend une vis sans fin rotative (146) avec des dents (148) conçue pour mettre en prise le motif de filetage, et dans lequel la rotation de la vis sans fin entraîne la rotation de la plate-forme autour de l'axe de tangage.

5. Système selon la revendication 1, dans lequel la base comprend une paire de bras arqués espacés (116) et dans lequel la fourche entre en prise et se déplace le long des bras arqués lors d'une rotation autour de l'axe de roulis.

6. Système selon la revendication 5, dans lequel au moins un des bras arqués de la base comprend un motif de filetage (118) formé dans une surface de celle-ci, et dans lequel l'actionneur de roulis comprend une vis sans fin rotative (142) avec des dents conçue pour mettre en prise le motif de filetage, et dans lequel la rotation de la vis sans fin entraîne la rotation de la fourche autour de l'axe de roulis.

7. Système selon la revendication 2, dans lequel l'actionneur du roulis, l'actionneur de tangage, l'actionneur de direction X et l'actionneur de direction Y présentent chacun des extrémités libres (141a à 141 d) respectives qui s'étendent vers l'extérieur à partir du châssis le long d'une direction commune.

8. Système selon la revendication 1, lequel comprend :
une unité de commande à distance (400, 800) comprenant une pluralité de commandes ;
et
une pluralité de câbles de commande (150a - 150d), chaque câble étant raccordé fonctionnellement à une commande respective et à un actionneur respectif, dans lequel le mouvement d'une commande fait fonctionner un actionneur respectif à l'aide d'un câble de commande respectif.

9. Système selon la revendication 8, dans lequel l'unité de commande comprend une commande de réglage du roulis (402a), une commande de réglage du tangage (402b), une commande de réglage de la direction X (402c) et une commande de réglage de la direction Y (402d) ;
dans lequel un câble de commande de roulis (150a) est raccordé fonctionnellement à la commande de réglage de roulis et à l'actionneur de roulis, dans lequel un mouvement de la commande de réglage de roulis fait fonctionner l'actionneur de roulis à l'aide du câble de commande de roulis ;
dans lequel un câble de commande de tangage (150b) est raccordé fonctionnellement à la commande de réglage du tangage et à l'actionneur du tangage, dans lequel un mouvement de la commande de réglage du tangage fait fonctionner l'actionneur du tangage à l'aide du câble de commande de tangage ;
dans lequel un câble de commande de la direction X (150c) est raccordé fonctionnellement à la commande de la direction X et à l'actionneur de la direction X, dans lequel un mouvement de la commande de réglage de la direction X fait fonctionner l'actionneur de la direction X à l'aide du câble de commande de la direction X ; et
dans lequel un câble de commande de la direction Y (150d) est raccordé fonctionnellement à la commande de la direction Y et à l'actionneur de la direction Y, dans lequel un mouvement de la commande de réglage de la direction Y fait fonctionner l'actionneur de la direction Y à l'aide du câble de commande de la direction Y.

10. Système selon la revendication 8, dans lequel les câbles de commande sont des câbles compatibles avec une IRM.

11. Système selon la revendication 1, comprenant en outre une sonde d'imagerie vidéo (500) fixée de manière amovible au châssis.

12. Système selon la revendication 1, comprenant en outre un élément tubulaire allongé (204) s'étendant à travers la plate-forme et la fourche et fixée à la table X-Y, dans lequel la canule d'acheminement est fixée de manière coulissante à l'intérieur de l'élément tubulaire et est mobile entre les positions déployées et rétractées, et dans lequel la canule d'acheminement est conçue pour translater en réponse au mouvement de translation de la table de support X-Y et tourner en réponse au mouvement de rotation de la fourche et de la plate-forme pour définir différentes trajectoires axiales qui se prolongent dans l'ouverture d'accès au patient.

13. Système selon la revendication 1, comprenant en outre une pluralité de repères d'alignement visibles par IRM (117) fixés au châssis.
